(19) Europäisches Patentamt / European Patent Office / Office européen des brevets

(11) **EP 2 961 315 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**14.12.2022 Patentblatt 2022/50**

(45) Hinweis auf die Patenterteilung:
**27.11.2019 Patentblatt 2019/48**

(21) Anmeldenummer: **14703383.1**

(22) Anmeldetag: **10.02.2014**

(51) Internationale Patentklassifikation (IPC):
**A61B 5/053** (2021.01)    **A61M 16/00** (2006.01)
**A61B 5/08** (2006.01)

(52) Gemeinsame Patentklassifikation (CPC):
**A61B 5/0536; A61M 16/024;** A61M 2016/0027;
A61M 2205/502; A61M 2230/08; A61M 2230/65

(86) Internationale Anmeldenummer:
**PCT/EP2014/052529**

(87) Internationale Veröffentlichungsnummer:
**WO 2014/131605 (04.09.2014 Gazette 2014/36)**

(54) **SYSTEM ZUR AUTOMATISIERTEN EINSTELLUNG EINES DURCH EINE BEATMUNGSEINRICHTUNG VORGEGEBENEN DRUCKS**

SYSTEM FOR AUTOMATED ADJUSTMENT OF A PRESSURE SPECIFIED BY A BREATHING DEVICE

SYSTÈME DE RÉGLAGE AUTOMATIQUE D'UNE PRESSION PRÉDÉFINIE PAR UN SYSTÈME D'ASSISTANCE RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.02.2013 DE 102013203177**

(43) Veröffentlichungstag der Anmeldung:
**06.01.2016 Patentblatt 2016/01**

(73) Patentinhaber: **Hamilton Medical AG
7402 Bonaduz (CH)**

(72) Erfinder:
• **NOVOTNI, Dominik
CH-7000 Chur (CH)**
• **LAUBSCHER, Thomas
CH-7403 Rhäzüns (CH)**

(74) Vertreter: **Schmitt-Nilson Schraud Waibel Wohlfrom
Patentanwälte Partnerschaft mbB
Pelkovenstraße 143
80992 München (DE)**

(56) Entgegenhaltungen:
DE-A1-102006 018 199    US-A- 5 746 214
US-A1- 2006 260 611    US-A1- 2007 246 046
US-A1- 2010 228 143

• BIKKER IDO G ET AL: "Bedside measurement of changes in lung impedance to monitor alveolar ventilation in dependent and non-dependent parts by electrical impedance tomography during a positive end-expiratory pressure trial in mechanically ventilated intensive care unit patients", CRITICAL CARE, BIOMED CENTRAL LTD., LONDON, GB, Bd. 14, Nr. 3, 30. Mai 2010 (2010-05-30), Seite R100, XP021085476, ISSN: 1364-8535, DOI: 10.1186/CC9036 in der Anmeldung erwähnt
• MOULOUD A DENAI ET AL: "Absolute Electrical Impedance Tomography (aEIT) Guided Ventilation Therapy in Critical Care Patients: Simulations and Future Trends", IEEE TRANSACTIONS ON INFORMATION TECHNOLOGY IN BIOMEDICINE, IEEE SERVICE CENTER, LOS ALAMITOS, CA, US, Bd. 14, Nr. 3, 1. Mai 2010 (2010-05-01), Seiten 641-649, XP011345687, ISSN: 1089-7771, DOI: 10.1109/TITB.2009.2036010
• Dräger: Gebrauchsanweisung PulmoVista 500, October 2010 (2010-10), pages 1-140,
• ECKHARD TESCHNER et al.: Elektrische Impedanztomographie: Von der Idee zur Anwendung des regionalen Beatmungsmonitorings, 2011, pages 1-128,

- Eduardo L. V. Costa et al: "Bedside estimation of recruitable alveolar collapse and hyperdistension by electrical impedance tomography", Intensive Care Medicine, vol. 35, no. 6, 3 March 2009 (2009-03-03), pages 1132-1137, Berlin, DE ISSN: 1432-1238
- Zhao Zhanqi et al: "PEEP titration guided by ventilation homogeneity: a feasibility study using electrical impedance tomography", Critical Care, vol. 14, no. 1, 30 January 2010 (2010-01-30), page R8, GB ISSN: 1364-8535
- Zhanqi Zhao et al: "Evaluation of an electrical impedance tomography-based global inhomogeneity index for pulmonary ventilation distribution", Intensive Care Medicine, vol. 35, no. 11, 4 August 2009 (2009-08-04), pages 1900-1906, Berlin, DE ISSN: 1432-1238, DOI: 10.1007/s00134-009-1589-y
- Inez Frerichs et al: "Thoracic Electrical Impedance Tomographic Measurements During Volume Controlled Ventilation-Effects of Tidal Volume and Positive End-Expiratory Pressure", IEEE Transactions on Medical Imaging, vol. 18, no. 9, 1 September 1999 (1999-09-01), US ISSN: 0278-0062
- Dräger, Kurven und Loops in der Beatmung, Frank Rittner, Martin Döring (D12, see pdf in DFR UA)
- Drägermedical, Evita 2 dura, Intensivtheraptie-Ventilator, Gebrauchsanweisung, Software 4.a (D13, see pdf in DFR UA)
- Dräger, Beatmungsmodi in der Intensivmedizin, Karin Deden, (D14, see pdf in DFR UA)

**Beschreibung**

Die vorliegende Erfindung betrifft ein System zur automatisierten Einstellung eines durch eine Beatmungseinrichtung vorgegebenen Drucks, insbesondere eines positiven endexpiratorischen Drucks und/oder eines maximalen Atemwegsdrucks. Die Erfindung betrifft auch eine Einrichtung zur maschinellen Beatmung, die über ein solches System zur Einstellung des vorgegebenen Drucks, insbesondere des positiven endexpiratorischen Drucks und/oder des maximalen Atemwegsdrucks, verfügt.

Bei heute gängigen Formen der maschinellen Beatmung wird dem Patienten Atemgas mit Überdruck zugeführt. Deswegen ist bei der Beatmung der Atemwegsdruck bzw. alveoläre Druck zumindest während der Inspirationsphase größer als der Druck im die Lungenbläschen bzw. Alveolen umgebenden Pleuraspalt. Während der Expirationsphase erfolgt keine Druckbeaufschlagung des Atemwegs durch die Beatmungseinrichtung, mit der Folge, dass das Lungengewebe sich entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Expirationsphase derart ungünstig einstellen, dass es zu einem Kollaps von Teilen der Alveolen kommt. Dann muss der kollabierte Teil des Lungenvolumens im nachfolgenden Atmungszyklus erst wieder von Neuem entfaltet werden. Die funktionelle Residualkapazität der Lunge wird stark beeinträchtigt, so dass die Sauerstoffsättigung abnimmt, und auch das Lungengewebe nimmt dauerhaft Schaden.

Um einen Kollaps von Alveolen am Ende der Expirationsphase zu verhindern, wird bei der maschinellen Überdruckbeatmung in der Regel ein sog. positiver endexpiratorischer Druck, in der Regel kurz als PEEP bezeichnet, eingestellt. Mit dieser Maßnahme kann in vielen Fällen eine Verbesserung der Sauerstoffsättigung erreicht werden.

Bei Beatmung mit PEEP beaufschlagt die Beatmungseinrichtung den Atemweg dauerhaft - also sowohl während der Inspirationsphase als auch während der Expirationsphase - mit einem vorbestimmten Überdruck, dem PEEP. Der PEEP liegt also auch nach dem Ende der Expirationsphase noch an. Der maximale Atemwegsdruck liegt am Ende der Inspirationsphase an, wenn der Atemweg durch das Beatmungsgerät am stärksten beaufschlagt wird.

Idealerweise sollte der PEEP genügend groß eingestellt werden, so dass während der Expirationsphase der alveoläre Druck nicht, oder jedenfalls nur so weit, unterhalb dem Druck im Pleuraspalt liegt, dass das alveoläre Gewebe unter der Wirkung des Drucks im Pleuraspalt nicht kollabiert.

Andererseits kann sich ein zu hoher Wert des PEEP negativ auswirken, insbesondere während der Inspirationsphase. Denn das Lungengewebe kann bei sehr hohen Atemwegsdrücken während der Inspirationsphase überdehnt werden. Aus der erforderlichen Beschränkung des maximalen Atemwegsdrucks am Ende der Inspirationsphase auf Werte, bei denen noch keine Überdehnung des Lungengewebes eintritt, ergibt sich bei hohem PEEP zudem eine Einschränkung für den möglichen Tidaldruck, d.h. Druckdifferenz zwischen dem Druck bei Expiration und dem maximalen Druck bei Inspiration.

Zahlreiche Studien deuten darauf hin, dass ein hoher Wert des PEEP den Rückfluss von venösem Blut zum Herzen behindern kann mit entsprechend negativen Auswirkungen auf das Herz-Kreislaufsystem.

In der klinischen Praxis werden Drücke wie PEEP oder maximal möglicher Tidaldruck bzw. maximaler Atemwegsdruck von Ärzten bzw. Pflegepersonal von Intensivstationen aufgrund vorgegebener physiologischer Parameter eines Patienten bzw. anhand von bekannten therapeutischen Richtwerten wie den sogenannten "ARDSnet-Richtlinien" eingestellt, siehe beispielsweise hinsichtlich der Einstellung eines geeigneten PEEP The Acute Respiratory Distress Syndrome Network, The New England Journal of Medicine, 2000, 342: S. 1301-1308 oder Grasso et al., American Journal of Respiratory Critical Care, 2007, 176: S. 761-767. Eine solche Einstellung erfolgt in der Regel im Vorhinein und wird nur sporadisch durch Ärzte oder Pflegepersonal angepasst.

Vorgegebene Richtlinien können naturgemäß nicht den tatsächlichen Zustand eines Patienten reflektieren, sondern geben Erfahrungswerte wieder. Man ist aber bestrebt, die Einstellung der die Beatmung bestimmenden Drücke wie PEEP oder maximaler Atemwegsdruck für jeden Patienten individuell anhand des aktuellen Zustands des Patienten vorzunehmen.

Bekannte Verfahren zur Ableitung von Information hinsichtlich geschlossener oder überdehnter Alveolen nehmen eine beträchtliche Zeit in Anspruch, während der eine Beatmung des Patienten in einem regelmäßigen Atmungszyklus nicht möglich ist. Dies ist etwa bei mit Hilfe der "Super-Syringe-Methode" aufgenommenen statischen Druck/Volumen Kurven (P/V-Kurven) der Fall, wie sie beispielsweise von Brochard L., Critical Care, 2006, 10: S. 156-158 beschrieben werden. Außerdem treten bei der Beatmung nicht selten inhomogene Lungenzustände auf, bei denen in einzelnen Bereichen der Lunge ein Verschließen von Alveolen zu beobachten ist, in anderen Bereichen hingegen nicht. Es ist sogar möglich, dass bei unveränderten Beatmungsparametern über einen Atmungszyklus hinweg in einigen Bereichen der Lunge die Alveolen sich während der Expirationsphase verschließen, während in anderen Bereichen der Lunge die Alveolen während der Inspirationsphase überdehnt werden. Die bekannten Verfahren können derartige Zustände nicht auflösen.

Grundsätzlich steht mit der beispielsweise in Bikker, I. G. et al., Critical Care, 2010, 14(3), R100; Tanaka, H., et al., American Journal of Respiratory and Critical Care, 2004, 169(7), S. 791 - 800 oder US 6 502 984 B1 beschriebenen Methode der elektrischen Impedanztomographie (EIT) ein Verfahren zur Verfügung, das es erlaubt, in Echtzeit Information über den Zustand der Lunge, insbesondere über das Öffnen und Schließen von Alveolen oder eine Überdehnung von Alveolen, zu erhalten. Die EIT ist ein nicht invasives Verfahren, das direkt am Patientenbett durchgeführt werden kann, und liefert räumlich differenzierte Information hinsichtlich unterschiedlicher Bereiche der Lunge. Mittels EIT ist es möglich, Bereiche der Lunge mit pathologischen Zuständen von "normal arbeitenden" Bereichen zu unterscheiden.

Beispielsweise schlägt die EP 1 593 341 B1 ein auf EIT basierendes Verfahren zur regionalen Bestimmung des alveolären Öffnens und des alveolären Schließens der Lunge vor. Hierzu soll ausgenutzt werden, dass die durch Atembewegungen des Patienten beeinflusste Änderung eines mittels EIT gewonnenen Impedanzsignals in Bereichen, in denen die Lunge noch nicht kollabiert ist, größer ist als in Bereichen mit kollabierten Alveolen. Zur Bestimmung der durch Atembewegungen hervorgerufenen Änderung des EIT-Impedanzsignals kann beispielsweise der Abstand zwischen Minima und Maxima im EIT-Impedanzsignal herangezogen werden.

Wegen ihrem großen Potential zur Gewinnung von zeitlich und räumlich aufgelöster Information hinsichtlich des Lungenzustands sind zahlreiche Anstrengungen unternommen worden, die EIT als Diagnoseinstrument zur Beurteilung des Verlaufs von Lungenkrankheiten nutzbar zu machen. Es besteht auch der Wunsch, solche Informationen bei der maschinellen Beatmung einsetzen zu können.

Die US 2010/0228143 A1 beschreibt eine Vorrichtung und ein Verfahren zur Bestimmung von funktionalen Eigenschaften der Lunge eines Patienten auf Grundlage von EIT-Signalen. Die EIT Signale geben eine räumliche Verteilung der elektrischen Impedanz in einem Schnitt durch den Thorax des Patienten wieder. Aus den Impedanzsignalen für die verschiedenen über die thorakale Schnittebene verteilten EIT-Pixeln wird eine über den gesamten Schnitt integrierte globale elektrische Impedanz bestimmt. Anhand des zeitlichen Verlaufs der globalen elektrischen Impedanz wird ein Atmungszyklus identifiziert und in verschiedene zeitliche Phasen unterteilt. Zusätzlich wird die thorakale Schnittebene in mehrere Bereiche aufgeteilt und in jeder Phase des Atmungszyklus für jeden Bereich jeweils das Verhältnis der elektrischen Impedanz des Unterbereichs zur jeweiligen globalen elektrischen Impedanz bestimmt. Anhand der so gewonnenen zeitlichen Verläufe des Impedanzverhältnisses für die einzelnen Bereiche kann die intratidale Gasverteilung über die Lunge, d.h. der Beitrag einzelner Bereiche der Lunge, zur Atmung im Verlauf eines Beatmungszyklus bestimmt werden. Es wird vorgeschlagen, anhand dieser Informationen auch verschiedene Beatmungsparameter, z.B. den PEEP oder das Tidalvolumen, festzulegen.

MOULOUD A DENAI ET AL: "Absolute Electrical Impedance Tomography (aEIT) Guided Ventilation Therapy in Critical Care Patients: Simulations and Future Trends", IEEE TRANSACTIONS ON INFORMATION TECHNOLOGY IN BIOMEDI-CINE, IEEE SERVICE CENTER, LOS ALAMITOS, CA, US, Bd. 14, Nr. 3, 1. Mai 2010 (2010-05-01), Seiten 641-649, XP011345687, ISSN: 1089-7771, DOI: 10.1109/TITB.2009.2036010 zeigt, dass ein EIT Verfahren eine Möglichkeit bietet, durchgehend Einstellungen eines Beatmungsgeräts von Intensivpatienten zu führen und diese Einstellungen vorzunehmen. MOULOUD zeigt das Prinzip der künstlichen Beatmung, Lungenaufrichtung, und EIT Tomographie an einem physiologischen Modell, welches ein Model der Respirationsmechanik, ein Modell der absoluten Widerstands der menschlichen Lunge als eine Funktion von Luftinhalt, und ein 2D finites Elemente Raster des Brustkorbs kombiniert, um eine EIT Bildrekonstruktion während der künstlichen Beatmung zu simulieren.

US 2006/260611 A1 zeigt eine Vorrichtung und ein Verfahren, um auf einfache Weise die Änderung der funktionellen Residualkapazität (FRC) zu ermitteln. Hierzu wird, ausgehend von einer ersten Beatmungsphase für maschinelle Beatmung, während einer zweiten Beatmungsphase ein Recruitment-Manöver durchgeführt und während einer dritten Beatmungsphase auf die maschinelle Beatmung zurückgeschaltet. Während der ersten Beatmungsphase und der dritten Beatmungsphase werden Referenzwert $U_{ref/1}$, $U_{ref/3}$ aus den end-exspiratorischen Werten der Impedanz-Messsignale U gebildet, wobei die Differenz $\Delta U$ ($\Delta FRC$) zwischen dem Referenzwert $U_{ref/3}$ der dritten Beatmungsphase und dem Referenzwert $U_{ref/1}$ der ersten Beatmungsphase ein Maß für die Änderung der funktionellen Residualkapazität der Lunge des Probanden ist.

Trotz aller Untersuchungen und Anstrengungen hinsichtlich der Nutzbarmachung von EIT zur Diagnose des Lungenzustands sind bis zum heutigen Tag allerdings keine Systeme zur Steuerung bzw. Regelung von bei maschineller Beatmung im Sinne einer geschlossenen Regelung weitgehend ohne Eingriff des Menschen zu steuern.

Der vorliegenden Erfindung liegt die Aufgabe zugrunde, ein System anzugeben, das die Möglichkeit der ganz oder jedenfalls weitgehend automatischen patientenbezogenen Einstellung bzw. Nachstellung eines durch die Beatmungseinrichtung vorgegebenen Drucks ermöglicht. Der durch die Beatmungseinrichtung vorgegebene Druck kann beispielsweise der positive endexpiratorische Druck sein. Zusätzlich oder alternativ soll es möglich sein, den maximalen Atemwegsdruck oder einen anderen von der Beatmungseinrichtung vorgegebenen Druck ganz oder jedenfalls weitgehend automatisch patientenbezogen einzustellen bzw. nachzustellen. Insbesondere soll die Einstellung bzw. Anpassung des durch die Beatmungseinrichtung vorgegebenen Drucks bei möglichst geringem Eingriff in den den Atmungszyklus oder Beatmungszyklus (beide Begriffe werden im Folgenden synonym gebraucht) erfolgen können und möglichst keine oder jedenfalls nur geringfügige Eingriffe durch Ärzte oder Pflegepersonal erforderlich machen.

Zur Lösung dieser Aufgabe wird erfindungsgemäß ein System zur automatisierten Einstellung eines durch eine Beatmungseinrichtung vorgegebenen Drucks gemäß Anspruch 1 vorgeschlagen. Das System umfasst eine Anordnung für elektrische Impedanztomographie (EIT) zur Erfassung einer elektrischen Impedanzverteilung entlang wenigstens eines zweidimensionalen Schnitts durch den menschlichen Thorax wenigstens am Ende einer Inspirationsphase und am Ende einer zugeordneten Expirationsphase; eine Einrichtung zur Unterteilung der erfassten elektrischen Impedanzverteilung am Ende der Inspirationsphase und am Ende der Expirationsphase in eine Mehrzahl von EIT-Pixeln und zur Bestimmung eines einem jeweiligen EIT-Pixel zugeordneten Werts der elektrischen Impedanz am Ende der Inspirationsphase und am Ende der Expirationsphase; und eine Einrichtung zur automatisierten Einstellung des durch die Beatmungseinrichtung vorgegebenen Drucks auf Grundlage eines Vergleichs (i) eines Unterschieds zwischen dem einem einzelnen EIT-Pixel zugeordneten Wert der elektrischen Impedanz am Ende der Inspirationsphase und dem dem jeweiligen einzelnen EIT-Pixel zugeordneten Wert der elektrischen Impedanz am Ende der Expirationsphase (ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz und am Ende der Expirationsphase.

Grundlage der automatisierten Bestimmung von durch die Beatmungseinrichtung vorgegebenen Drücken, wie beispielsweise dem maximalen Atemwegsdruck (im Folgenden auch kurz als Paw_max bezeichnet) und/oder dem positiven endexpiratorischen Druck (im Folgenden auch kurz als PEEP bezeichnet), ist das Ergebnis einer durch elektrische Impedanztomographie (im Folgenden auch kurz als EIT bezeichnet) gewonnenen Verteilung der elektrischen Impedanz über mindestens einen zweidimensionalen Schnitt durch den Thorax eines Patienten. Solche Daten können mittels bekannter EIT-Anordnungen in nicht invasiver Weise und in Echtzeit am Patientenbett gewonnen werden. Die EIT-Daten werden in der Regel anhand von Messungen des elektrischen Potentials an einer Mehrzahl von um den Thorax in Umfangsrichtung verteilt angeordneten Elektroden in Reaktion auf das Anlegen einer Potentialdifferenz zwischen je zwei benachbarten der Elektroden durch Rückprojektionsalgorithmen bestimmt. Bei modernen EIT-Geräten erfolgt die Berechnung der Impedanzverteilung automatisiert anhand von numerischen Algorithmen. Die Impedanzverteilung liegt dann in Form von numerisch berechneten EIT-Werten vor, die jeweils einem aus einer Mehrzahl von in der thorakalen Schnittebenene angeordneten EIT-Pixeln zugeordnet sind. Die EIT-Pixel bilden ein die thorakale Schnittebene abdeckendes regelmäßiges oder unregelmäßiges Raster aus. Wenn die EIT-Daten in anderer Form, etwa als analoge Verteilung der Impedanz über die thorakale Schnittebene vorliegen, kann eine entsprechende Rasterung vorgenommen werden. Die aus der Rasterung erhaltene EIT Pixelverteilung kann unmittelbar als Eingangsgröße zur automatischen Bestimmung des Beatmungsdrucks bzw. von Beatmungsdrücken herangezogen werden.

Es ist auch denkbar, die Rasterung entsprechend einer Verteilung bestimmter Zonen in der thorakalen Schnittebene vorzunehmen, beispielsweise Regionen die bestimmten Organen zugeordnet sind, von anderen Regionen zu unterscheiden oder aber nur grob zwischen eher dorsal gelegenen und eher ventral gelegenen Bereichen bei supiner Lagerung eines Patienten zu unterscheiden.

Im Folgenden wird der Einfachheit halber von einem Raster aus EIT-Pixeln über einen zweidimensionalen thorakalen Schnitt hinweg ausgegangen. Es sei darauf hingewiesen, dass die beschriebenen Gedanken zur Auswertung der EIT-Daten auch auf EIT-Vorrichtungen anwendbar sind, bei denen mehrere Schnittebenen durch den Thorax parallel abgetastet werden können und damit dreidimensionale EIT-Bilder erzeugt werden können, wie beispielsweise in US 6 501 198 B1 beschrieben.

Die Erfindung folgt dem Grundgedanken, die durch EIT gelieferten Impedanzdaten pixelweise auszuwerten. Hierzu werden die einem einzelnen EIT-Pixel zugeordneten Impedanzwerte jeweils am Ende der Inspiration und am Ende der zugeordneten Expiration miteinander verglichen und der sich so ergebende Unterschied wiederum mit einem für die Gesamtheit der Pixel repräsentativen Unterschied zwischen der Impedanz am Ende der Inspiration und der Impedanz am Ende der zugeordneten Expiration verglichen. Der Vergleich ist so gestaltet, dass anhand des so gewonnenen Vergleichsergebnisses eine angezeigte Einflussnahme auf einen bei maschineller Beatmung vorzugebenden Druck, insbesondere auf den maximalen Atemwegdruck Paw_max und/oder den positiven endexpiratorischen Druck PEEP, abgeleitet werden kann. Diese Vorgehensweise erlaubt damit eine weitgehend von menschlichen Eingriffen unbeeinflusste automatische Einstellung bzw. Nachführung des gewünschten Beatmungsdrucks durch das Beatmungsgerät.

Beispielsweise kann der angesprochene Unterschied zwischen jeweils einzelnen EIT-Pixeln zugeordneten Impedanzwerten am Ende der Inspiration und am Ende der zugeordneten Expiration und/oder der angesprochene für die Gesamtheit der Pixel repräsentative Unterschied zwischen der Impedanz am Ende der Inspiration und der Impedanz am Ende der zugeordneten Expiration in Form einer Differenz zwischen den jeweiligen Impedanzwerten am Ende der Inspiration und der am Ende der Expiration definiert sein. Alternativ ist es auch denkbar, den angesprochenen Unterschiede als ein Verhältnis zwischen den jeweiligen Impedanzwerten am Ende der Inspiration und am Ende der Expiration zu definieren. Differenzbildung und Verhältnisbildung kann man auch kombinieren, falls gewünscht. Es ist auch denkbar, noch andere Größen heranzuziehen, solange diese ein Maß für die relative Änderung des jeweiligen Impedanzwerts zwischen Ende Inspiration und Ende Expiration darstellen.

Der angesprochene Vergleich ist in Form eines "ja/nein" Tests ausgestaltet. Damit ist gemeint, dass anhand des Vergleichs geprüft wird, ob eine vorbestimmte Bedingung erfüllt ist, oder ob diese Bedingung nicht erfüllt ist. Auf diese Weise liefert der Vergleich ein einfach zu handhabendes Kriterium zur Entscheidung, ob seitens des Beatmungsgeräts auf den gewünschten Beatmungsdruck Einfluss zu nehmen ist, d.h. der jeweils eingestellte Druck erhöht oder abgesenkt werden muss, oder ob dieser Druck unverändert bleiben kann.

Das jeweilige Ende einer Inspirationsphase bzw. das zugeordnete Ende einer Expirationsphase kann anhand eines einzigen Atmungszyklusses bestimmt sein. Es ist aber auch möglich, einen Impedanzverlauf über einen Atmungszyklus hinweg zu Grunde zu legen, der sich aus mehreren Atmungszyklen durch geeignete Mittelwertbildung und/oder Integration ergibt.

Gemäß einer Ausführungsform kann der auf Grundlage der Gesamtheit der EIT-Pixel bestimmte Unterschied zwischen dem Wert der elektrischen Impedanz am Ende der Inspirationsphase und dem Wert der elektrischen Impedanz am Ende der Expirationsphase ein Maximalwert aller Unterschiede zwischen dem Wert der elektrischen Impedanz am Ende der Inspirationsphase für eines der EIT-Pixel und dem Wert der elektrischen Impedanz am Ende der Expirationsphase für dieses EIT-Pixel sein. Der Vergleich liefert dann eine Aussage, ob die einem jeweiligen EIT-Pixel zugeordnete Impedanzänderung während eines Atmungszyklusses gegenüber der maximalen Impedanzänderung in der thorakalen Schnittebene während dieses Atmungszyklusses eher klein, eher groß oder eher durchschnittlich ist. Anhand solcher Aussagen lässt sich aus Erfahrungswerten bzw. durch Expertensysteme recht einfach und sicher ableiten, inwieweit auf den Beatmungsdruck Einfluss zu nehmen ist.

In weiteren Ausführungsformen kann zusätzlich vorgesehen sein, dass die Einrichtung zur automatisierten Einstellung des durch die Beatmungseinrichtung vorgegebenen Drucks den einem jeweiligen EIT-Pixel zugeordneten Unterschied zwischen dem Wert der elektrischen Impedanz am Ende der Inspirationsphase und dem Wert der elektrischen Impedanz am Ende der Expirationsphase auch mit dem jeweils auf Grundlage der Gesamtheit der EIT-Pixel bestimmten Wert der elektrischen Impedanz am Ende der Inspirationsphase und/oder am Ende der Expirationsphase vergleicht. Ein solcher Vergleich mit dem Wert der elektrischen Impedanz am Ende der Inspirationsphase liefert eine Aussage darüber, ob die einem jeweiligen EIT-Pixel zugeordnete Impedanzänderung während eines Atmungszyklusses zu einem Pixel in der thorakalen Schnittebene gehört, bei dem der maximale Atemwegsdruck eher klein, eher groß oder eher durchschnittlich ist. Dementsprechend liefert ein Vergleich mit dem Wert der elektrischen Impedanz am Ende der Expirationsphase eine Aussage darüber, ob die einem jeweiligen EIT-Pixel zugeordnete Impedanzänderung während eines Atmungszyklusses zu einem Pixel in der thorakalen Schnittebene gehört, bei dem der am Ende der Expirationsphase verbleibende Atemwegsdruck eher klein, eher groß oder eher durchschnittlich ist. Damit lässt sich recht eindeutig unterscheiden, ob eine anhand der Impedanzänderung zwischen Impedanz am Ende der Inspirationsphase und Impedanz am Ende der Expirationsphase angezeigte Einwirkung auf den Beatmungsdruck eher auf den maximalen Atemwegsdruck und/oder auf den positiven endexpiratorischen Druck anzuwenden ist.

In besonderen Ausführungsformen kann der angesprochene pixelweise Vergleich derart implementiert sein, dass die Einrichtung zur automatisierten Einstellung des durch die Beatmungseinrichtung vorgegebenen Drucks anhand des Vergleichs diejenigen EIT-Pixel bestimmt, die eine vorgegebene Bedingung erfüllen, und dann eine Korrektur des vorgegebenen Drucks vornimmt, wenn der Anteil der EIT-Pixel, die die Bedingung erfüllen, größer ist als ein vorgegebener Schwellenwert. Damit wird im Wesentlichen der Anteil von EIT-Pixeln an der Gesamtzahl von EIT-Pixeln in the thorakalen Schnittebene bestimmt, der auf einen physiologisch unerwünschten Zustand schließen lässt. Es ist dabei denkbar, die einzelnen EIT-Pixel unterschiedlich groß zu wählen oder bei der Berechnung des Anteils unterschiedlich stark zu gewichten. Die Bedingung kann anhand physiologischer Erkenntnisse festgelegt sein. Wenn die Bedingung erfüllt ist, deutet dies auf einen physiologisch unerwünschten Zustand hin, z.B. auf ein Kollabieren von Alveolen während der Expirationsphase, oder auf ein Überdehnen von Alveolen während der Inspirationsphase.

Für den Fall, dass der durch die Beatmungseinrichtung vorgegebene positive endexpiratorische Druck (PEEP) automatisch eingestellt bzw. nachgeführt werden soll, kann beispielsweise auf Grundlage eines Vergleichs(i) eines Unterschieds zwischen dem einem einzelnen EIT-Pixel zugeordneten Wert der elektrischen Impedanz am Ende der Inspirationsphase und dem dem jeweiligen einzelnen EIT-Pixel zugeordneten Wert der elektrischen Impedanz am Ende der Expirationsphase einerseits,(ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz am Ende der Expirationsphase sowie mit dem auf Grundlage der Gesamtheit der EIT-Pixel bestimmten Wert der elektrischen Impedanz am Ende der Expirationsphase andererseits der Anteil von EIT-Pixeln in der thorakalen Schnittebene, die in mehr als vernachlässigbarem Maß kollabierte Alveolen enthalten, bestimmt werden. Ist dieser Anteil oberhalb eines vorbestimmten ersten Schwellenwerts, kann man davon ausgehen, dass der PEEP zu niedrig eingestellt ist, und durch Erhöhen des PEEP der Gasaustausch in der Lunge verbessert werden kann.

Beispielsweise kann der auf Grundlage der Gesamtheit der EIT-Pixel bestimmte Wert der elektrischen Impedanz am Ende der Expirationsphase ein Minimalwert aller Werte der elektrischen Impedanz am Ende der Expirationsphase für ein jeweiliges der EIT-Pixel sein. Man nimmt also das EIT-Pixel mit der geringsten Impedanz als Bezugsgröße und bestimmt aus allen EIT-Pixeln eine Untermenge von EIT-Pixeln, welche die geringste Impedanz aufweisen. Diese Untermenge von EIT Pixeln enthält im Wesentlichen alle möglichen Kandidaten für EIT-Pixel mit kollabierten Alveolen. Anhand des für die Untermenge weiters vorgesehen pixelweisen Vergleichs der Impedanzänderung über einen Atmungszyklus eines jeweiligen EIT-Pixels gegenüber der auf Grundlage aller EIT-Pixel bestimmten Impedanzänderung kann relativ genau auf diejenigen EIT-Pixel geschlossen werden, die kollabierte Alveolen enthalten.

Als Ergebnis von Vergleichen der beschriebenen Art, kann die Einrichtung zur automatisierten Einstellung eines durch die Beatmungseinrichtung vorgegebenen positiven endexpiratorischen Drucks diejenigen EIT-Pixel, für die der Vergleich positiv ist, Lungenbereichen mit kollabierten Alveolen zuordnen."Positiv" soll in diesem Zusammenhang bedeuten, dass die jeweiligen EIT-Pixel eine vorgegebene Bedingung erfüllen, die als charakteristisch für kollabierte Alveolen angesehen wird.

In besonderen Ausführungsfomen kann die Zuordnung von EIT-Pixeln zu Lungenbereichen mit kollabierten Alveolen dann erfolgen, wenn die folgenden beiden Bedingungen erfüllt sind:

$$\text{EIT\_ei\_xy} - \text{EIT\_ee\_xy} < k1 * \max(\text{EIT\_ei\_xy} - \text{EIT\_ee\_xy};$$
$$\text{für alle möglichen xy})$$

UND

$$\text{EIT\_ee\_xy} < k2 * \min(\text{EIT\_ee\_xy}; \text{für alle möglichen xy})$$

mit:

EIT_ei_xy:     einem jeweiligen EIT Pixel zugeordneter elektrischer Impedanzwert am Ende der Inspirationsphase
EIT_ee_xy:     einem jeweiligen EIT Pixel zugeordneter elektrischer Impedanzwert am Ende der Expirationsphase,
$0 \leq k1 \leq 1$,     insbesondere $0{,}3 \leq k1 \leq 0{,}7$, insbesondere $0{,}4 \leq k1 \leq 0{,}6$, insbesondere $k1 = 0{,}5$;
$k2 \geq 1$,     insbesondere $1{,}0 \leq k2 \leq 1{,}6$, insbesondere $1{,}2 \leq k2 \leq 1{,}4$, insbesondere $k2 = 1{,}3$.

Eine elegante automatische Einstellung bzw. Nachführung des positiven endexpiratorischen Drucks ergibt sich dann, wenn die Beatmungseinrichtung für den Fall, dass der Anteil von EIT-Pixeln, für die der Vergleich positiv ist, an der Gesamtzahl der EIT-Pixel einen vorbestimmten ersten Schwellenwert übersteigt, den Wert des positiven endexpiratorischen Drucks für die nachfolgenden Atmungszyklen um einen vorbestimmten Betrag erhöht. Der zweite Schwellenwert kann zwischen 5 und 25 % liegen, insbesondere 10 % betragen. Zur erstmaligen Einstellung eines Werts für den PEEP geht man dann von einem vergleichsweise niedrigen PEEP-Wert aus (z.B. 10 cm $H_2O$) und lässt die Beatmungseinrichtung Schritt für Schritt den PEEP erhöhen (z.B. um 1 cm $H_2O$ pro Schritt), bis der Anteil an kollabierten Alveolen während der Expiration unter den ersten Schwellenwert sinkt. Der PEEP wird anschließend stabil gehalten, bis erneut festgestellt wird, dass der Anteil an kollabierten Alveolen während der Expiration den ersten Schwellenwert erreicht oder übersteigt. In Reaktion wird der PEEP erneut schrittweise höher gewählt, bis wieder der erste Schwellenwert unterschritten wird. Der Vergleich kann dabei während der Beatmung des Patienten in in vorbestimmten zeitlichen Abständen wiederholt werden und der positive endexpiratorische Druck für die nachfolgenden Atmungszyklen solange erhöht werden, bis der Anteil von EIT-Pixeln, für die der Vergleich positiv ist, an der Gesamtzahl der EIT-Pixel unterhalb des ersten Schwellenwerts liegt. Generall wird man bei dieser von niedrigem PEEP Niveau ausgehenden automatischen Einstellung bwz. Nachführung des PEEP auch eine Obergrenze für den PEEP festlegen, und zwar insbesondere so, dass kein Barotrauma während der Beatmung entsteht. Es kann dabei ausreichen, einen als sicher geltenden oberen Grenzwert für den PEEP manuell vorzugeben, z.B. dass der PEEP immer kleiner bleiben soll als 25 cm $H_2O$.

Außerdem kann vorgesehen sein, dass der Wert des ersten Schwellenwerts mit zunehmendem Wert des PEEP größer gewählt wird. Das führt dazu, dass mit steigendem Niveau des PEEP eine weitere Erhöhung nur dann stattfindet, wenn ein immer größerer Anteil der EIT-Pixel kollabierte Alveolen aufweist. Auf diese Weise kann man sicherstellen, dass der Wert des PEEP nicht auf ewig erhöht wird, wenn der Anteil an EIT-Pixeln mit kollabierten Alveolen sich mit zunehmendem PEEP nicht oder nur geringfügig verringert. Beispielsweise kann man mit jeder Erhöhung des PEEP den ersten Schwellenwert um einen Betrag zwischen 0,3 und 3 % erhöhen, insbesondere um 1 % erhöhen.

Für den Fall, dass zusätzlich oder alternative zum positiven endexpiratorischen Druck der durch die Beatmungseinrichtung vorgegebene maximalen Atemwegsdruck Paw_max automatisch eingestellt bzw. nachgestellt werden soll, kann beispielsweis auf Grundlage eines Vergleichs(i) eines Unterschieds zwischen dem einem einzelnen EIT-Pixel zugeordneten Wert der elektrischen Impedanz am Ende der Inspirationsphase und dem dem jeweiligen einzelnen EIT-Pixel zugeordneten Wert der elektrischen Impedanz am Ende der Expirationsphase einerseits,(ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz und am Ende der Expirationsphase sowie mit dem auf Grundlage der Gesamtheit der EIT-Pixel bestimmten Wert der elektrischen Impedanz am Ende der Inspirationsphase andererseits der Anteil von EIT-Pixeln in der thorakalen Schnittebene, die mehr als in vernachlässigbarem Maß überdehnte Alveolen aufweisen, bestimmt werden. Ist dieser Anteil oberhalb einer vorgegebenen zweiten Anteilsschwelle, die unabhängig von der ersten Anteilsschwelle zur Einstellung des PEEP ist, kann man davon ausgehen, dass der maximale Atemwegsdruck zu hoch eingestellt ist und durch Verringern des maximalen Atemwegsdrucks das Lungengewebe geschont werden kann. Die Verringerung des maximalen Atemwegsdrucks kann - bei unverändertem PEEP - über eine Verringerung des maximalen Tidaldrucks erreicht werden. Sofern es angezeigt ist, in den folgenden Atmungszyklen einen geringeren Wert für den PEEP zu wählen, kann die Verringerung des PEEP bei der Verringerung des maximalen Atemwegsdrucks berücksichtigt werden, so dass der Einfluss auf den maximalen Tidaldruck wenigstens zum Teil kompensiert wird.

Beispielsweise kann der auf Grundlage der Gesamtheit der EIT-Pixel bestimmte Wert der elektrischen Impedanz am Ende der Inspirationsphase ein Maximalwert aller Werte der elektrischen Impedanz am Ende der Inspirationsphase für ein jeweiliges der EIT-Pixel sein. Das bedeutet, dass man das EIT-Pixel in the thorakalen Schnittebene mit der höchsten Impedanz als Bezugsgröße heranzieht, um aus allen EIT-Pixeln eine weitere Untermenge von EIT-Pixeln zu bestimmen, welche die höchste Impedanz am Ende der Inspirationsphase aufweisen. Etwaige EIT-Pixel mit überdehnten Alveolen sollten in dieser weiteren Untermenge enthalten sein, so dass lediglich innerhalb dieser Untermenge nach denjenigen Pixeln gesucht werden muss, bei denen tatsächlich Überdehnung von Alveolen aufgetreten ist. Führt man den vorgeschlagenen pixelweisen Vergleich der Impedanzänderung zwischen Ende Inspiration und Ende Expiration für alle EIT-Pixel dieser Untermenge durch, kann man relativ genau diejenigen EIT-Pixel identifizieren, die überdehnte Alveolen enthalten.

Ähnlich wie oben beschrieben kann die Einrichtung zur automatisierten Einstellung eines durch die Beatmungseinrichtung vorgegebenen maximalen Atemwegsdrucks diejenigen EIT-Pixel, für die der Vergleich positiv ist, Lungenbereichen mit überdehnten Alveolen zuordnen."Positiver Vergleich" heißt wiederum, dass die jeweiligen EIT-Pixel eine vorgegebene Bedingung erfüllen, die als charakteristisch für eine Überdehnung von Alveolen angesehen wird.

In Ausführungsbeispielen kann die Zuordnung von EIT-Pixeln zu Lungenbereichen mit überdehnten Alveolen dann erfolgen, wenn die folgenden beiden Bedingungen erfüllt sind:

$$EIT\_ei\_xy - EIT\_ee\_xy < k3 * max(EIT\_ei\_xy - EIT\_ee\_xy;$$
$$\text{für alle möglichen xy})$$

UND

$$EIT\_ei\_xy > k4 * max(EIT\_ei\_xy; \text{für alle möglichen xy})$$

mit:

EIT_ei_xy: einem jeweiligen EIT Pixel zugeordneter elektrischer Impedanzwert am Ende der Inspirationsphase
EIT_ee_xy: einem jeweiligen EIT Pixel zugeordneter elektrischer Impedanzwert am Ende der Expirationsphase,
$0 \leq k3 \leq 1$, insbesondere $0,3 \leq k3 \leq 0,7$, insbesondere $0,4 \leq k3 \leq 0,6$, insbesondere $k3 = 0.5$;
$0 \leq k4 \leq 1$, insbesondere $0,5 \leq k4 \leq 0,9$, insbesondere $0,6 \leq k4 \leq 0,8$, insbesondere $k4 = 0,7$.

Auch für den maximalen Atemwegsdruck lässt sich eine elegante automatische Einstellung bzw. Nachführung erreichen, wenn die Einrichtung zur automatisierten Einstellung eines durch die Beatmungseinrichtung vorgegebenen maximalen Atemwegsdrucks dann, wenn der Anteil von EIT-Pixeln, für die der Vergleich positiv ist, an der Gesamtzahl der EIT-Pixel einen vorbestimmten zweiten Schwellenwert übersteigt, den Wert des maximalen Atemwegsdrucks für die nachfolgenden Atmungszyklen um einen vorbestimmten Betrag verringert. Der zweite Schwellenwert kann in einem Bereich zwischen 5 und 25 % liegen, insbesondere 10 % betragen. Zur erstmaligen Einstellung eines Werts für den maximalen Atemwegsdruck geht man dann von einem vergleichsweise hohen Wert für maximalen Atemwegsdruck aus (z.B. 40 cm $H_2O$) und lässt die Beatmungseinrichtung Schritt für Schritt den maximalen Atemwegsdruck verringern (z.B. um 1 cm $H_2O$ pro Schritt), bis der Anteil an während der Inspiration überdehnten Alveolen den zweiten Schwellenwert unterschreitet. Der maximale Atemwegsdruck wird anschließend stabil gehalten, bis erneut festgestellt wird, dass der Anteil während der Inspiration überdehnter Alveolen wieder den zweiten Schwellenwert erreicht oder diesen übersteigt. In Reaktion wird der maximale Atemwegsdruck erneut schrittweise verringert, bis wieder der zweite Schwellenwert unterschritten wird. Der Vergleich kann dabei während der Beatmung des Patienten in vorbestimmten zeitlichen Abständen wiederholt werden und der maximale Atemwegsdruck für die nachfolgenden Atmungszyklen solange verringert werden, bis der Anteil von EIT-Pixeln, für die der Vergleich positiv ist, an der der Gesamtzahl der EIT-Pixel unterhalb des zweiten Schwellenwerts liegt. Der Anfangswert für den maximalen Atemwegsdruck kann manuell vorgegeben werden. Generell wird man einen Wert auswählen, der zwar über dem optimal einzustellenden Wert liegt, aber noch niedrig genug ist, dass bei Beatmung über wenige Atmungszyklen hinweg noch keine ernsthaften Gewebeschäden an der Lunge des Patienten auftreten.

Ein üblicher Wert für den anfänglichen maximalen Atemwegsdruck beträgt beispielsweise 40 cm $H_2O$.

Weiterhin kann auch bei der Einstellung des maximalen Atemwegsdrucks vorgesehen sein, dass der Wert des zweiten Schwellenwerts mit abnehmendem Wert des maximalen Atemwegsdrucks größer gewählt wird. Diese Maßnahme stellt sicher, dass die Beatmungseinrichtung den Wert des maximalen Atemwegsdrucks nicht bis auf Null verringert, wenn in einem Ausnahmefall der Anteil an EIT-Pixeln mit überdehnten Alveolen sich mit abnehmendem maximalem Atemwegsdruck nicht oder nur geringfügig verringert. Vielmehr wird die Zunahme des zweiten Schwellenwerts mit abnehmendem maximalen Atemwegsdruck so gewählt, dass sich auf alle Fälle ein moderat oberhalb des PEEP liegender maximaler Atemwegsdruck einstellt. Beispielsweise kann man mit jeder Verringerung des maximalen Beatmungsdrucks den zweiten Schwellenwert um einen Betrag zwischen 0,3 und 3 % erhöhen, insbesondere um 1 % erhöhen.

In weiteren Ausführungsformen kann die Anordnung für elektrische Impedanztomographie (EIT) eine elektrische Impedanzverteilung in Form von EIT-Pixeln, die in einem vorgegebenen Raster angeordnet sind, zugeordneten elektrischen Impedanzwerten erfassen. Für den Fall, dass die EIT-Anordnung bereits ein geeignet gerastertes Bild von Impedanzwerten liefert, kann dann die Einrichtung zur Unterteilung der erfassten elektrischen Impedanzverteilung in eine Mehrzahl von EIT-Pixeln einem jeweiligen Rasterelement genau den jeweiligen Wert der elektrischen Impedanz zuordnen. Alternativ kann auch die vom EIT gelieferte Rasterung in Form von EIT-Elementarpixeln in eine andere gewünschte Rasterung in Form von die thorakale Schnittebene abbildenden EIT-Pixeln umgerechnet werden und jedem der EIT-Pixel ein entsprechender Impedanzwert zugeordnet werden.

Häufig sind die von der EIT-Anordnung erfassen EIT-Signale von höherfrequenten Signalanteilen überlagert, die nichts mit dem Atmungszyklus bzw. Beatmungszyklus zu tun haben. Ein Beispiel sind kardiale Einflüsse, weil auch die Tätigkeit des Herz-Kreislauf-Systems die Impedanz in einigen Bereichen der thorakalen Schnittebene beeinflusst. Da kardiale Einflüsse einer signifikant höheren Taktfrequenz unterliegen, können derartige Störeinflüsse durch eine Filtereinrichtung ausgefiltert werden.

Die Filtereinrichtung ist beispielsweise ein Tiefpassfilter oder Bandpassfilter mit geeigneter Grenzfrequenz, das in den Signalweg der EIT Signale geschaltet ist. Eine Beatmungseinrichtung mit entsprechender Tiefpassfunktion zum Ausfiltern von kardialen Einflüssen ist beispielsweise aus der DE 103 01 202 B3 bekannt.

Die vorliegende Erfindung bezieht sich auch auf eine Einrichtung zur maschinellen Beatmung, welche ein ein System zur automatischen Einstellung eines von der Beatmungseinrichtung vorgegebenen Drucks mit wenigstens einem der vorangehend beschriebenen Merkmale umfasst. Die Beatmungseinrichtung kann dabei zur dauerhaften Beatmung von Patienten, z.B. in Intensivstationen, ausgebildet sein und insbesondere die Einstellung der angesprochenen Drücke weitgehend automatisiert, bei nur sporadischen Eingriffen durch Ärzte oder Pflegepersonal vornehmen.

In einem weiteren Aspekt betrifft die vorliegende Erfindung ein Verfahren zur automatisierten Einstellung eines durch eine Beatmungseinrichtung vorgegebenen Drucks, insbesondere eines positiven endexpiratorischen Drucks (PEEP) und eines maximalen Atemwegsdrucks (Paw_max), bei welchem mit Hilfe einer Anordnung für elektrische Impedanztomographie (EIT) eine elektrische Impedanzverteilung entlang wenigstens eines zweidimensionalen Schnitts durch den menschlichen Thorax wenigstens am Ende einer Inspirationsphase und am Ende einer zugeordneten Expirationsphase erfasst wird, die erfasste elektrische Impedanzverteilung am Ende der Inspirationsphase und am Ende der Expirationsphase in eine Mehrzahl von EIT-Pixeln unterteilt wird und einem jeweiligen EIT-Pixel ein Wert der elektrischen Impedanz am Ende der Inspirationsphase und am Ende der Expirationsphase zugeordnet wird, und der durch die Beatmungseinrichtung vorgegebene Druck auf Grundlage eines Vergleichs (i) eines Unterschieds zwischen dem einem einzelnen EIT-Pixel zugeordneten Wert der elektrischen Impedanz am Ende der Inspirationsphase und dem dem jeweiligen einzelnen EIT-Pixel zugeordneten Wert der elektrischen Impedanz am Ende der Expirationsphase (ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz und am Ende der Expirationsphase bestimmt wird. Ein derartiges Verfahren kann insbesondere nach einem oder mehrenen der oben ausgeführten weiterbildenden Merkmale ausgestaltet sein.

Die Erfindung wird nachfolgend unter Bezugnahme auf die Zeichnungen anhand von Ausführungsbeispielen im Detail erläutert. Es zeigt:

Fig. 1: In stark vereinfachter und schematisierter Darstellung die wesentlichen Elemente einer Beatmungseinrichtung mit EIT-Anordnung zur Erfassung von elektrischen Impedanzsignalen entlang eines thorakalen Schnitts;

Fig. 2a: Eine schematisierte Darstellung von in Form eines regelmäßigen Gitters von EIT-Pixeln gerasterten elektrischen Impedanzwerten über den thorakalen Schnitt aus Fig. 1 am Ende der Inspirationsphase bei gesunder Lunge;

Fig. 2b: Eine schematisierte Darstellung von in Form eines regelmäßigen Gitters von EIT-Pixeln gerasterten elektrischen Impedanzwerten über den thorakalen Schnitt aus Fig. 1 am Ende der zugeordneten Expirationsphase bei einer gesunden Lunge;

Fig. 2c: Eine schematisierte Darstellung der gerasterten EIT-Pixel aus Fig. 2a und 2b, wobei EIT-Pixel, bei denen die Impedanzdifferenz zwischen Ende Inspirationsphase und Ende Expirationsphase mindestens so groß ist wie das 0,5 fache der maximalen Impedanzdifferenz zwischen Ende Inspirationsphase und Ende Expirationsphase unter allen EIT-Pixeln in dem thorakalen Schnitt, dunkel schattiert sind und alle EIT-Pixel mit einer entsprechenden Impedanzdifferenz kleiner als das 0,5 fache der maximalen Impedanzdifferenz hell schattiert sind;

Fig. 2d: Eine schematisierte Darstellung der gerasterten EIT-Pixel aus Fig. 2a und 2b, wobei EIT-Pixel mit Impedanzwerten am Ende der Inspirationsphase, die größer sind als das 0,7fache der maximalen Impedanz am Ende der Inspirationsphase unter allen EIT-Pixeln in dem thorakalen Schnitt, dunkel schattiert sind und Pixel mit Impedanzwerten höchstens so groß wie das 0,7fache der maximalen Impedanz hell schattiert sind;

Fig. 2e: Eine schematisierte Darstellung der gerasterten EIT-Pixel aus Fig. 2a und 2b, wobei EIT-Pixel mit Impedanzwerten am Ende der Expirationsphase, die mindestens gleich dem 1,3fachen der minimalen Impedanz am Ende der Expirationsphase unter allen EIT-Pixeln in dem thorakalen Schnitt sind, dunkel schattiert sind und Pixel mit Impedanzwerten kleiner als das 1,3 fache der minimalen Impedanz hell schattiert sind;

Fig. 3a - 3e: Ansichten entsprechend Fig. 2a - 2e, aber für eine Lunge mit während der Expirationsphase kollabierten Alveolen; und

Fig. 4a - 4e: Ansichten entsprechend Fig. 2a - 2e, aber für eine Lunge mit während der Inspirationsphase überdehnten Alveolen.

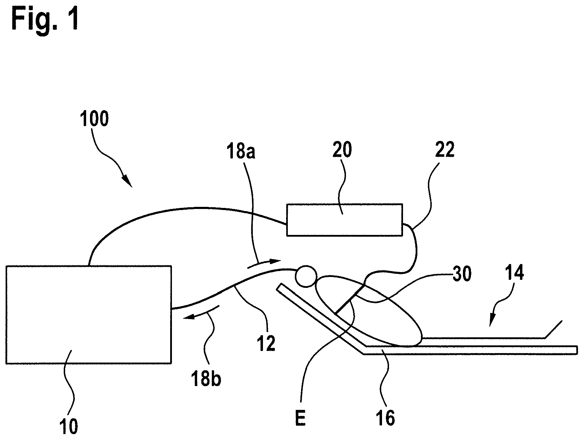

Fig. 1 zeigt in stark vereinfachter Darstellung und in Form eines Blockdiagramms schematisiert die wesentlichen Elemente einer Beatmungseinrichtung 100, die ein Beatmungsgerät 10 zur Erzeugung von Beatmungsgasflüssen bzw. Beatmungsdrücken und zur Steuerung der Beatmung sowie eine elektrische Impedanz (EIT)-Anordnung 20 umfasst. Die EIT-Anordnung 20 dient zur Erfassung von Werten der elektrischen Impedanz im Gewebe eines schematisch mit 14 umrissenen Patienten entlang eines in Fig. 1 mit E angedeuteten sich in diesem Beispiel von dorsal nach ventral erstreckenden Schnitts durch den Thorax des beatmeten Patienten 14. Die EIT-Anordnung (20) errechnet aus den erfassten Daten Bildern, die den Verlauf der elektrischen Impedanz über den thorakalen Schnitt E hinweg wiedergeben.

Die Beatmungseinrichtung 100 ist in Fig. 1 in einem Zustand mit über einen Beatmungsschlauch 12 intubierter Luftröhre (Trachea) des beatmeten Patienten 14 gezeigt. Der Patient 14 liegt in supiner Haltung auf einem Krankenbett 16, in der Regel in einer Intensivstation. Der Tubus der Beatmungseinrichtung 100 ist, in der Regel über die nicht gezeichnete Mundöffnung des Patienten, ein Stück weit in die Trachea eingeschoben, um den Atemweg während einer Inspirationsphase des Atmungszyklus mit Beatmungsgas zu beaufschlagen. Ausgeatmete Luft wird während einer sich an die Inspirationsphase anschließenden Expirationsphase ebenfalls über den Tubus abgeleitet, der sich an seinem mit dem Beatmungsgerät 10 verbundenen Ende in ein erstes Ende und ein zweites Ende verzweigt. Das erste Ende des Tubus ist über ein Atemwegeingangsventil mit einem Atemwegeingangsanschluss des Beatmungsgeräts 10 zur Beaufschlagung mit Beatmungsgas unter dem Inspirationsdruck PInsp verbunden. Das zweite Ende des Tubus ist über ein Atemwegausgangsventil mit einem Atemwegausgangsanschluss des Beatmungsgeräts 10 zur Beaufschlagung des Expirationsdrucks PExp verbunden. Atemwegeingangsventil und Atemwegausgangsventil sind während des sich zyklisch wiederholenden Atmungszyklus abwechselnd geöffnet um den Beatmungsschlauch 12 jeweils mit dem Inspirationsdruck PInsp oder dem Expirationsdruck PExp zu beaufschlagen.

Sowohl der Inspirationsdruck PInsp als auch der Expirationsdruck PExp werden von dem Beatmungsgerät 10 nach vorgegebenen zeitlichen Mustern erzeugt, um sich periodisch wiederholende Atmungszyklen zu erzeugen. In jedem Atmungszyklus strömt einzuatmendes Beatmungsgas während der Inspirationsphase in Richtung der Lunge des Patienten 14, wie durch den Pfeil 18a in Fig. 1 angedeutet. Während der nachfolgenden Expirationsphase strömt das auszuatmende Atemgas von der Lunge des Patienten zum Beatmungsgerät 10 zurück, wie durch den Pfeil 18b angedeutet. Normalerweise bleibt während der Inspirationsphase das Atemwegeingangsventil geöffnet und der Atemwegeingang wird mit dem Inspirationsdruck PInsp - dieser ist in der Regel größer als der Expirationsdruck PExp - beaufschlagt. Während der Expirationsphase ist das Atemwegeingangsventil 18 geschlossen und das Atemwegausgangsventil ist geöffnet. Dann ist der Atemwegeingang mit dem Expirationsdruck PExp beaufschlagt.

Im Zusammenhang mit der vorliegenden Erfindung sind jegliche Formen bekannter Beatmungsmuster einsetzbar, beispielsweise druckkontrollierte Beatmungsformen, volumenkontrollierte Beatmungsformen oder auch Beatmungsformen, bei denen druckkontrollierte und volumenkontrollierte Aspekte kombiniert sind. Neben rein maschinenkontrollierten Beatmungsformen, bei denen der zeitliche Verlauf des Inspirationsdrucks Plnsp und ggf. auch des Expirationsdrucks PExp von dem Beatmungsgerät 10 bestimmt werden, sind auch Beatmungsformen denkbar, in denen Spontanatmungsbemühungen des Patienten entweder die maschinelle Beatmung unterstützten können oder die maschinelle Beatmung zur Unterstützung von Spontanatmungsbemühungen des Patienten dient. In solchen Beatmungsformen werden der zeitliche Verlauf von Inspirationsdruck Plnsp bzw. Expirationsdruck PExp sowie häufig ebenfalls die Stellung von Eingangsventil bzw. Ausgangsventil nicht allein von dem Beatmungsgerät 10 vorgegeben, sondern von Spontanatmungsbemühungen des Patienten mit beeinflusst.

Das Atemgas kann Umgebungsluft enthalten, wird aber in der Regel einen vorbestimmten Anteil reinen Sauerstoff, sehr häufig als $FiO_2$ bezeichnet, enthalten, der über dem Sauerstoffanteil der Umgebungsluft liegt. Das Atemgas wird in der Regel außerdem befeuchtet sein.

Der Fluss des Atemgases am Atemwegeingang wird mit Hilfe eines nicht gezeigten Atemwegeingangsflusssensors bestimmt. Der Atemwegeingangsflusssensor beruht auf der Erfassung einer Druckdifferenz dP zwischen einem Eingangsvolumen und einem mit dem Eingangsvolumen in Verbindung stehenden Ausgangsvolumen, und liefert eine Bestimmung des Atemgasmassenflusses am Atemwegeingang. Aus dem Drucksignal im Ausgangsvolumen lässt sich zugleich recht einfach der Wert des Atemwegeingangsdrucks Paw ableiten. Der Atemwegseingangsdruck Paw ist der Druck, mit dem die Lunge während der Inspiration beaufschlagt wird. Dieser Druck ist etwas höher als der in der Lunge tatsächlich herrschende Beatmungsdruck. In der Regel wird bei maschineller Beatmung in jedem Atmungszyklus der Beatmungsdruck während der Inspirationsphase von einem niedrigen Startwert ausgehend rampenartig erhöht, bis am Ende der Inspirationsphase ein hoher Endwert erreicht wird, der ggf. eine gewisse Zeit beibehalten wird. In dieser Phase des Atmungszyklus stellt sich in der Lunge ein maximaler Atemwegsdruck Paw_max ein. Der maximale Atemwegsdruck Paw_max ließe sich grundsätzlich am Ende der Inspirationsphase am Atemwegeingang messen, sofern sich ein vollständiger Druckausgleich einstellt. Dann gäbe der am Ende der Inspirationsphase gemessene Atemwegeingangsdruck Paw in recht guter Näherung den maximalen Atemwegsdruck wieder. Da sich an die Inspirationsphase die Expirationsphase anschließt, wird ein solcher vollständiger Druckausgleich in der Praxis häufig nicht erreicht, es sei den man führt künstlich ein kurzzeitiges, ca. 1 - 5 s langes Okklusionsmanöver, d.h. Atemwegeingangsventil und Atemwegausgangsventil bleiben gleichzeitig geschlossen, am Ende der Inspirationsphase durch. Derartige Manöver unterbrechen jedoch den Atmungszyklus und werden daher während der Beatmung nicht regelmäßig ausgeführt. Während der Expirationsphase wird der Atemwegeingang nicht mit einem Tidaldruck beaufschlagt, sondern die in der Lunge befindliche Luft soll in Folge von Relaxationsbewegung des Lungengewebes aus der Lunge gedrückt werden. Häufig wird jedoch auch während der Expirationsphase der Beatmungsschlauch 12 und damit der Atemwegeingang mit einem positiven Druck beaufschlagt. Dieser Druck wird positiver endexpiratorischer Druck, oder kurz PEEP, genannt und soll verhindern, dass in manchen Bereichen der Lunge Alveolen während der Expirationsphase unter dem auf geschwächtes Lungengewebe einwirkenden Druck aus dem Thorax kollabieren. Bei Beatmung mit PEEP ergibt sich der den Beatmungsgasstrom letztliche treibende Tidaldruck als Differenz aus dem Beatmungsdruck und dem PEEP.

Der in den Alveolen der Lunge herrschende Druck hängt ab vom Atemwegeingangsdruck Paw sowie der Strömung des Atemgases in die Lunge bzw. aus der Lunge heraus. Im Falle eines Druckausgleichs zwischen Atemwegeingang und Alveolen ist der alveoläre Druck Palv gleich dem Atemwegeingangsdruck. Ein derartiger Druckausgleich hat zur Konsequenz, dass der Atemgasfluss zum Erliegen kommt. Beispielsweise kann ein kurzzeitiges (z.B. 1 bis 5s langes) Okklusionsmanöver zu einem Druckausgleich führen. Wenn der Gasfluss im Atemweg zum Erliegen kommt, kann der alveoläre Druck Palv mittels einer Bestimmung des Atemwegeingangsdrucks Paw bestimmt werden. Bei ununterbrochenen Atmungszyklen wird am Ende der Expirationsphase am Atemwegeingang zwar ein minimaler Wert des Drucks Paw gemessen. Dieser liegt aber normalerweise etwas über dem eingestellten PEEP.

Sowohl bei der physiologischen Atmung als auch bei maschineller Beatmung wird der Fluss des Atemgases bestimmt durch eine Druckdifferenz zwischen dem alveolären Druck Palv und dem Atemwegeingangsdruck Paw.

Im Falle der rein physiologischen Atmung wird zum Einatmen eine negative Druckdifferenz, d.h. ein Unterdruck, zwischen dem alveolären Druck Palv und dem Atemwegeingangsdruck Paw erzeugt durch Ausdehnung des Thorax und damit verbundener Absenkung des Drucks in dem zwischen Thorax und Lunge gebildeten Pleuraspalt. Die Ausatmung erfolgt passiv durch Entspannen des Thorax und elastischer Rückstellung des Lungengewebes. Aus diesem Grund ist bei physiologischer Atmung der Druck im Pleuraspalt immer kleiner als der alveoläre Druck.

Bei maschineller Beatmung wird das Atemgas mit Überdruck - dem positven Tidaldruck - in die Lunge gepumpt. Aus diesem Grund ist bei maschineller Beatmung während der Inspirationsphase der Atemwegeingangsdruck Paw = Plnsp größer als der alveoläre Druck Palv und dieser wiederum größer als der Druck im Pleuraspalt Ppl. Während der Expiration wird der Atemwegeingang mit einem Atemwegsdruck PExp beaufschlagt, der geringer ist also der alveoläre Druck Palv, so dass Atemgas aus den Alveolen herausströmt. Im Falle eines sehr kleinen Atemwegsdrucks PExp kann es vorkommen, dass am Ende der Expiration, wenn nur noch sehr wenig Gas in der Lunge vorhanden ist, der Druck im Pleuraspalt Ppl den alveolären Druck Palv in so hohem Maße übersteigt, dass ein Teil der Alveolen der Lunge kollabiert.

Das Kollabieren der Alveolen lässt sich verhindern, wenn man auch in der Expirationsphase den Atemwegeingang mit einem zusätzlichen positiven Druck beaufschlagt. Es liegt dann am Atemwegeingang dauerhaft, d.h. während der Inspirationsphase und auch während der Expirationsphase ein positiver Atemwegsdruck an. Dieser positive Atemwegsdruck wird als positiver endexpiratorischer Druck oder PEEP bezeichnet.

In Fig. 1 mit 20 ist eine zusätzliche Einrichtung für elektrische Impedanztomographie (EIT) bezeichnet. Die EIT-Einrichtung 20 ist mittels einer Kabelverbindung 22 mit einem Brustgurt 30 verbunden, der in Brusthöhe um den Thorax des Patienten geschnallt ist. Der Brustgurt 30 ist entlang seiner Länge mit in regelmäßigen Abständen angeordneten elektrischen Elektroden (nicht gezeigt) versehen. Die Elektroden liegen unmittelbar auf der Haut um den Thorax des Patienten 14 herum an. Somit kann die EIT-Einrichtung 20 über die Kabelverbindung 22 und die am Brustgurt 30 angeordneten Elektroden elektrischen Strom in den Körper des Patienten 14 leiten. Umgekehrt können die Elektroden am Brustgurt als Sensoren zur Erfassung von den Körper des Patienten 14 durchfließenden elektrischen Strömen dienen. Die EIT-Einrichtung 20 ist in der Lage, die Elektroden des Brustgurts 30 einzeln anzusteuern. Somit kann die EIT-Einrichtung 20 zwischen je zwei beliebigen - in der Regel aus Berechnungsgründen benachbarten - Elektroden des Brustgurts 30 eine elektrische Spannung anlegen. In Reaktion auf das Anlegen derartiger Spannungen fließen elektrische Ströme im Thorax des Patienten. Die EIT-Einrichtung 20 kann auch über die übrigen Elektroden am Brustgurt 30 diese in Reaktion auf das Anlegen der elektrischen Spannung im Thorax zwischen je zwei Elektroden des Brustgurts fließenden elektrischen Ströme rings um den Thorax erfassen und daraus räumlich aufgelöst entsprechende Werte der elektrischen Impedanz ableiten. Auf diese Weise lässt sich in Echtzeit und in nicht invasiver Weise eine Verteilung der elektrischen Impedanz in der durch den Brustgurt 14 festgelegten thorakalen Schnittebene E erfassen.

Anhand des mittels EIT gelieferten Impedanzsignals, bzw. anhand der räumlichen Verteilung der elektrischen Impedanz in der thorakalen Schnittebene, lässt sich auf das in der Lunge befindliche Atmungsgas schließen. Die Verteilung der elektrischen Impedanz in der thorakalen Schnittebene ist ein direktes Maß für in der Lunge befindliches Atmungsgas. Bereiche mit hohem Gasgehalt erscheinen in einem EIT-Bild als Werte hoher Impedanz, während Bereiche mit niedrigem Gasgehalt als Werte geringer Impedanz erscheinen. Anhand von räumlichen und/oder zeitlichen Veränderungen des mittels EIT gelieferten elektrischen Impedanzsignals lässt sich der Kollaps von Alveolen unter Druck von außen während der Expiration sowie eine Überdehnung von Alveolen unter Druck vom Atemwegeingang während der Inspiration im Prinzip erkennen. Von der EIT gelieferte Impedanzbilder können daher als Grundlage dienen, um bestimmte bei der maschinellen Beatmung vorzugebende Gasdrücke, insbesondere den PEEP und/oder den maximalen Atemwegsdruck, einzustellen. Dies kann manuell geschehen, indem ein Arzt oder eine Plfegekraft eine EIT-Messung durchführt, sich den dabei erhaltenen Impedanzverlauf in der thorakalen Schnittebene ansieht und dann die genannten Drücke entsprechend einstellt.

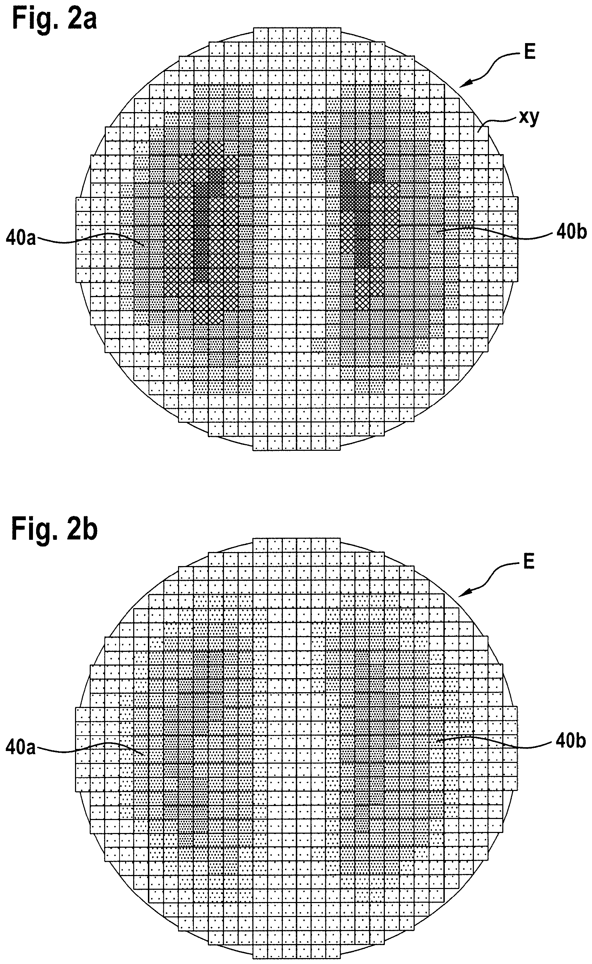

Die vorliegende Erfindung schlägt vor, die mittels EIT gelieferten räumlich und zeitlich aufgelösten Werte der elektrischen Impedanz als Grundlage zu einer weitgehend automatisierten, d.h. grundsätzlich ohne menschliche Eingriffe auskommenden Einstellung von für die maschinelle Beatmung wichtigen Drücken wie PEEP oder maximalem Atemwegsdruck Paw_max zu nehmen. Dies gelingt mit Hilfe einer ganz bestimmten Art und Weise der Auswertung der mittels EIT gelieferten Impedanzverteilungen. Diese Auswertung basiert auf einem Vergleich von einzelnen EIT-Pixeln in dem thorakalen Schnitt E zugeordneten Werten der elektrischen Impedanz mit einem Wert der elektrischen Impedanz, der eine charakteristische Eigenschaft der Gesamtheit aller EIT-Pixel in der thorakalen Schnittebene wiedergibt. Dieser Vergleich wird, EIT-Pixel für EIT-Pixel, für alle EIT-Pixel der thorakalen Schnittebene ausgewertet. Anhand der relativen Häufigkeit von EIT-Pixeln, die eine oder mehrere vorbestimmte Bedingungen erfüllen, lassen sich Kriterien dafür ableiten, ob beispielweise ein eingestellter Wert für den PEEP ausreichend hoch ist, um ein Kollabieren von Alveolen zu verhindern, und/oder ob ein eingestelter Wert für den maximalen Atemwegsdruck Paw_max niedrig genug ist, um ein Überdehnen von Alveolen zu verhindern. Das hinter dieser Idee stehende Prinzip soll im Folgenden anhand der Figuren 2a - 2e, 3a - 3e und 4a - 4e verdeutlicht werden.

In den Figuren 2a - 2e, 3a - 3e und 4a - 4e ist in vereinfachter Weise jeweils als Kreis die thorakale Schnittebene E angedeutet. Die thorakale Schnittebene E ist in ein gitterförmiges Muster aus einzelnen EIT-Pixeln unterteilt, die insgesamt die gesamte Schnittebene E innerhalb des Thorax ausfüllen. In Fig. 2a und 2b ist zur Verdeutlichung je eines der EIT Pixel mit xy bezeichnet. Man kann sagen, dieses EIT-Pixel befindet sich in Reihe x (in dem Beispiel Reihe 8 von oben gezählt) und in Spalte y (in dem Beispiel Spalte 28 von links gezählt), so dass sich alle EIT-Pixel in eindeutiger Weise durch ein Paar von Zahlenwerten x (= Reihe) und y (=Spalte) identifizieren lassen. Es sei angemerkt, dass eine derartige Anordnung von EIT-Pixeln xy unmittelbar von der EIT-Einrichtung 20 geliefert werden kann. Es ist aber genauso denkbar, eine Anordnung von EIT-Pixeln xy, wie in Fig. 2a - 2e gezeigt, aus einer beliebigen von der EIT-Einrichtung gelieferten Impedanzverteilung umzurechnen. Außerdem ist ein regelmäßiges Muster der EIT-Pixel wie in Fig. 2A - 2e gezeigt nicht zwingend. Die einzelnen EIT-Pixel xy können auch völlig unregelmäßig angeordnet sein und/oder völlig unterschiedliche Größen und/oder völlig unterschiedliche Formen aufweisen. Es sollten allerdings weitgehend keine von der Lunge des Patienten eingenommenen Bereiche der thorakalen Schnittebene E existieren, die keinem der EIT-Pixel xy zugeordnet sind.

Die Fig. 2a und 2b zeigen in schematischer Weise für jedes EIT-Pixel xy Impedanzwerte am Ende der Inspirationsphase EIT_ei_xy (Fig. 2a) und Impedanzwerte am Ende der zugehörigen Expirationsphase EIT_ee_xy (Fig. 2b), und zwar für eine gesunde Lunge 40. In den beiden Figuren ist der Wert der elektrischen Impedanz jeweils durch Grauwerte kodiert, wobei dunkle Stellen EIT-Pixeln xy mit hoher Impedanz EIT_ei_xy bzw. EIT_ee_xy und helle Stellen EIT-Pixeln xy mit geringer Impedanz EIT_ei_xy bzw. EIT_ee_xy entsprechen. Werte hoher elektrischer Impedanz zeigen Bereiche mit hohem Gehalt an Atemgas an, während Werte geringer Impedanz Bereiche mit niedrigem Gehalt an Atemgas anzeigen. Deutlich ist in Fig. 2a und 2b die Lunge 40 des Patienten 14 mit rechtem Lungenflügel 40a linkem Lungenflügel 40b zu erkennen.

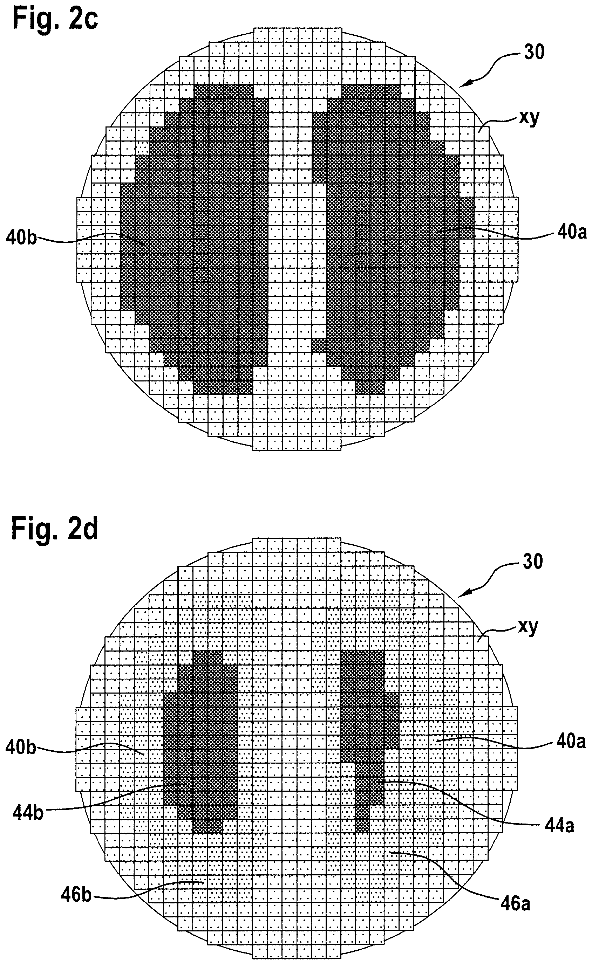

Anteile der EIT-Signale bzw. Impedanzwerte, die auf andere Organe als die Lunge 40 zurückzuführen sind, sind in Fig. 2a und 2b (ebenso wie in allen folgenden Figuren 2c - 2e, 3a - 3e, 4a - 4e) weggelassen. Beispielsweise können auf kardiale Einflüsse zurückzuführende EIT-Signalanteile anhand der gegenüber der Beatmungsfrequenz deutlich höheren Herzfrequenz durch Anwendung entsprechender Tief- oder Bandpassfilter aus den ursprünglich gewonnenen EIT-Signalen ausgefiltert werden, wie dies beispielsweise in DE 103 01 202 B1 beschrieben ist.

In Fig. 2a - am Ende der Inspirationsphase - sind die der Lunge 40 zugeordneten einzelnen EIT-Pixel xy generell dunkler schattiert als die jeweils zugehörigen EIT-Pixel xy in Fig. 2b - am Ende der Expirationsphase. Dem entspricht, dass am Ende der Inspirationsphase zumindest bei gesunder Lunge jeder Bereich der Lunge einen insgesamt höheren Atemgasanteil enthalten sollte als am Ende der Expirationsphase.

In relativ klarer Weise lässt sich dieser Befund der Fig. 2c entnehmen. Dort ist eine weitere schematisierte Darstellung der gerasterten EIT-Pixel xy aus Fig. 2a und 2b gezeigt. Allerdings ist in Fig. 2c für jedes EIT-Pixel xy der thorakalen Schnittebene E die Impedanzdifferenz zwischen der am Ende der Inspirationsphase erfassten Impedanz EIT_ei_xy und der am Ende der Expirationsphase erfassten Impedanz EIT_ee_xy dargestellt. Die Darstellung der Impedanzdifferenz in Fig. 2c ist dem Sinne "binärisiert", dass alle EIT Pixel xy, die eine vorbestimmte Bedingung erfüllen, dunkel schattiert sind, und alle anderen EIT-Pixel, die diese Bedingung nicht erfüllen, hell schattiert sind. Im Falle der Fig. 2c lautet die vorbestimmte Bedingung, dass dunkel schattiert alle EIT-Pixel xy sind, bei denen die genannte Impedanzdifferenz mindestens so groß ist wie das 0,5 fache der unter allen EIT-Pixeln xy in dem thorakalen Schnitt E auftretenden maximalen Impedanzdifferenz EIT_ei_xy - EIT_ee_xy zwischen Impedanz am Ende der Inspirationsphase EIT_ei_xy und Impedanz am Ende der Expirationsphase EIT_ee_xy. Formelmäßig ausgedrückt:
EIT Pixel xy sind dunkel schattiert, wenn gilt:

$$\text{EIT\_ei\_xy} - \text{EIT\_ee\_xy} \geq 0{,}5 * \max(\text{EIT\_ei\_xy} - \text{EIT\_ee\_xy};$$
$$\text{für alle möglichen xy}),$$

mit

EIT_ei_xy:    einem jeweiligen EIT-Pixel zugeordneter elektrischer Impedanzwert am Ende der Inspirationsphase
EIT_ee_xy:    einem jeweiligen EIT-Pixel zugeordneter elektrischer Impedanzwert am Ende der Expirationsphase.

Alle übrigen EIT-Pixel mit einer entsprechenden Impedanzdifferenz kleiner als das 0,5 fache der maximalen Impedanzdifferenz sind dagegen in Fig. 2c hell schattiert.

Man erkennt unschwer, dass in praktisch allen Bereichen der Lunge 40 die Impedanzdifferenz größer ist als der in diesem Beispiel festgelegte Schwellenwert. Da der Schwellenwert als die Hälfte der maximalen in dem thorakalen Schnitt E auftretenden Impedanzdifferenz zwischen dem Ende der Inspiration und dem Ende der Expiration festgelegt ist, folgt aus diesem Befund, dass für alle EIT-Pixel xy die Impedanzdifferenz nur um einen im Vergleich zur maximalen Impedanzdifferenz kleinen Wert streut. Das bedeutet, dass für alle Bereiche der Lunge ein deutlicher Unterschied zu erkennen ist hinsichtlich der Füllung von Alveolen mit Atemgas am Ende der Inspiration gegenüber dem am Ende der Expiration in den Alveolen Restvolumen. Dies ist ein starkes Indiz dafür, dass alle Bereiche der Lunge normal arbeiten und weder kollabierte noch überdehnte Alveolen auftreten.

Fig. 2d zeigt eine der Fig. 2c recht ähnliche Darstellung der thorakalen Schnittebene E mit gerasterten EIT-Pixeln xy. Fig. 2c stellt letztlich eine binärisierte Version der bereits in Fig. 2a gezeigten Impedanzverteilung am Ende der Inspiration dar. In Fig. 2c dunkel schattiert sind diejenigen EIT-Pixel xy aus Fig. 2a, welchen Impedanzwerte am Ende der Inspirationsphase EIT_xy_ei zugeordnet sind, die größer sind als das 0,7fache der unter allen EIT Pixeln xy in der thorakalen Schnittebene E am Ende der Inspirationsphase auftretenden maximalen Impedanz EIT_ei_max. Hell schattiert sind alle übrigen EIT-Pixel xy, denen Impedanzwerte am Ende der Inspirationsphase EIT_xy_ei zugeordnet sind, die höchstens so groß sind wie das 0,7fache der unter allen EIT Pixeln xy in der thorakalen Schnittebene E am Ende der Inspirationsphase auftretenden maximalen Impedanz EIT_ei_max. Formelmäßig ausgedrückt:

EIT-Pixel xy sind in Fig. 2d dunkel schattiert, wenn gilt:

$$EIT\_ei\_xy > 0,7 * EIT\_ei\_max,$$

EIT Pixel xy sind in Fig. 2d hell schattiert, wenn gilt:

$$EIT\_ei\_xy \leq 0,7 * EIT\_ei\_max,$$

mit: EIT_ei_max = max(EIT_ei_xy; für alle möglichen xy)

Man erkennt recht leicht in Fig. 2d jeweils einen zentralen Bereich 44a, 44b der beiden Lungenflügel 40a, 40b, in dem tendenziell hohe Atemgasdrücke am Ende der Inspiration herrschen und je einen peripheren Bereich 46a, 46b, in dem tendenziell niedrigere Atemgasdrücke am Ende der Inspiration herrschen. Wie Fig. 2c zeigt, ist in allen Bereichen der Lunge 40 jedoch die Impedanzdifferenz zwischen Ende der Inspiration und Ende der Expiration oberhalb des Schwellenwerts, was anzeigt, dass die Alveolen in allen Lungenbereichen normal am Gasaustausch teilnehmen unabhängig davon, ob am Ende der Inspiration ein hoher oder ein nur moderater Atemgasdruck herrscht.

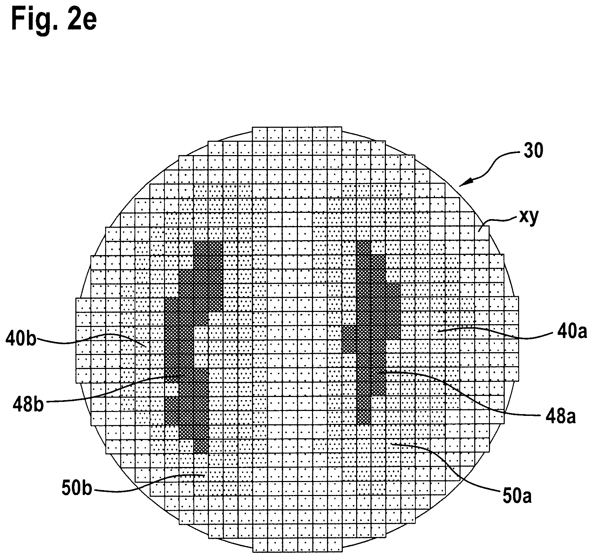

Fig. 2e zeigt eine weitere der Fig. 2d recht ähnliche Darstellung der thorakalen Schnittebene E mit gerasterten EIT-Pixeln xy. Fig. 2e stellt letztlich eine binärisierte Version der bereits in Fig. 2b gezeigten Impedanzverteilung am Ende der Expiration dar. In Fig. 2e dunkel schattiert sind diejenigen EIT-Pixel xy aus Fig. 2b, welchen Impedanzwerte am Ende der Expirationsphase EIT_ee_xy zugeordnet sind, die mindestens so groß sind wie das 1,3fache der unter allen EIT Pixeln xy in der thorakalen Schnittebene E am Ende der Expirationsphase auftretenden minimalen Impedanz EIT_ee_min. Hell schattiert sind alle übrigen EIT-Pixel xy, denen Impedanzwerte am Ende der Expirationsphase EIT_ee_xy zugeordnet sind, die kleiner sind als das 1,3 fache der unter allen EIT Pixeln xy in der thorakalen Schnittebene E am Ende der Expirationsphase auftretenden minimalen Impedanz EIT_ee_min. Formelmäßig ausgedrückt:

EIT Pixel xy sind in Fig. 2e dunkel schattiert, wenn gilt:

$$EIT\_ee\_xy \geq 1,3 * EIT\_ee\_min,$$

EIT Pixel xy sind in Fig. 2e hell schattiert, wenn gilt:

$$EIT\_ei\_xy < 1,3 * EIT\_ee\_min,$$

mit: EIT_ee_min = min(EIT_ee_xy; für alle möglichen xy)

Man erkennt in Fig. 2e, dass am Ende der Expiration in einem zentralen Bereich 48a, 48b der beiden Lungenflügel 40a, 40b tendenziell höhere Atemgasdrücke verbleiben als in einem peripheren Bereich 50a, 50b. Dennoch zeigt, Fig. 2c, dass in allen Bereichen der Lunge 40 die Differenz zwischen der Impedanz am Ende der Inspiration und der Impedanz am Ende der Expiration deutlich höher ist als der gewählte Schwellenwert, was anzeigt, dass die Alveolen in allen Lungenbereichen normal am Gasaustausch teilnehmen.

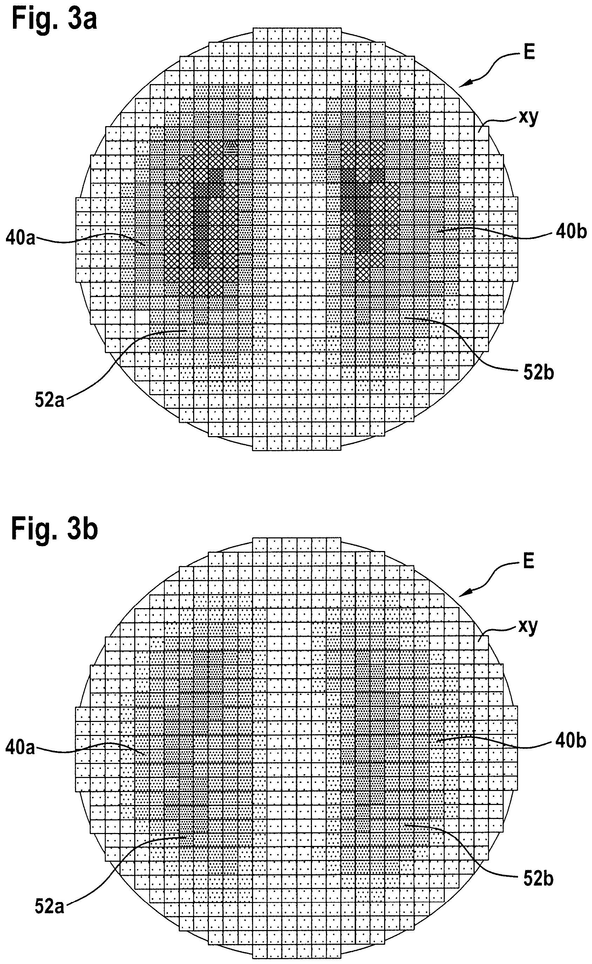

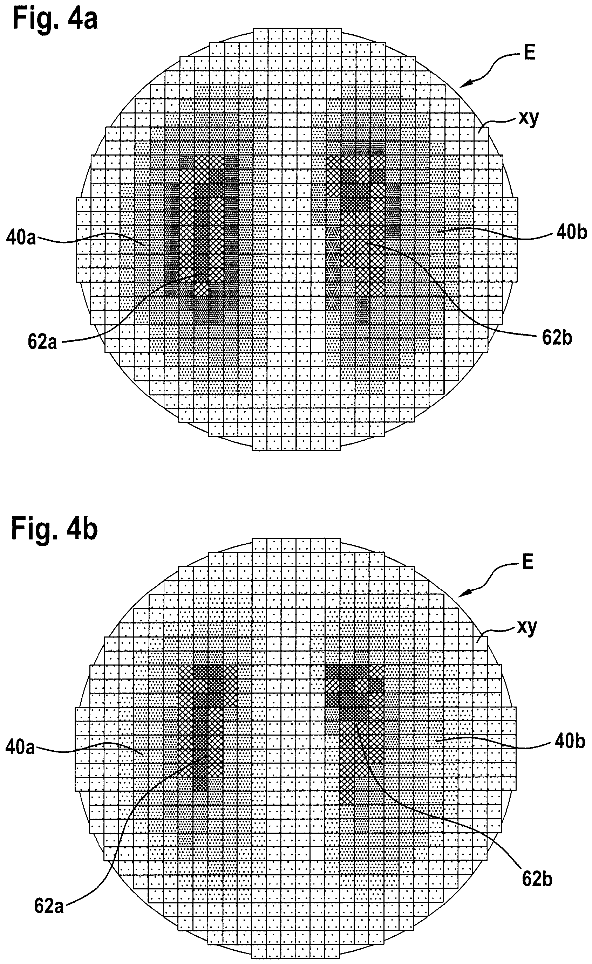

Die mit Bezug auf Fig. 2a - 2e für eine gesunde Lunge gezeigten Verhältnisse liegen anders im Falle einer Lunge, deren Alveolen infolge der Beaufschlagung mit zu niedrigem PEEP während der Expiration teilweise kollabiert sind, siehe Fig. 3a - 3e oder im Falle einer Lunge, deren Alveolen in Folge der Beaufschlagen mit überhöhtem Atemgasdruck während der Inspiration überdehnt wurden, siehe Fig. 4a - 4e. Die in Fig. 3a - 3e und Fig. 4a - 4e gezeigten Darstellungen entsprechen jeweils den Darstellungen in Fig. 2a - 2e, aber für eine Lunge mit während der Expiration teilweise kollabierten Alveolen bzw. für eine Lunge mit während der Inspirationsphase teilweise überdehnten Alveolen. In allen Figuren sind dieselben Bezugszeichen verwendet wie in Fig. 2a - 2e und es wird auf die jeweilige Beschreibung der bezeicheten Elemente zu Fig. 2a - 2e verwiesen, um Wiederholungen zu vermeiden.

Wie im Falle einer gesunden Lunge zeigt sich auch für die Lunge mit teilweise kollabierten Lungen (Fig. 3a und 3b) wie für die Lunge mit teilweise überdehnten Alveolen (Fig. 4a und 4b), dass die Impedanzwerte am Ende der Inspiration generell höher sind als am Ende der Expiration.

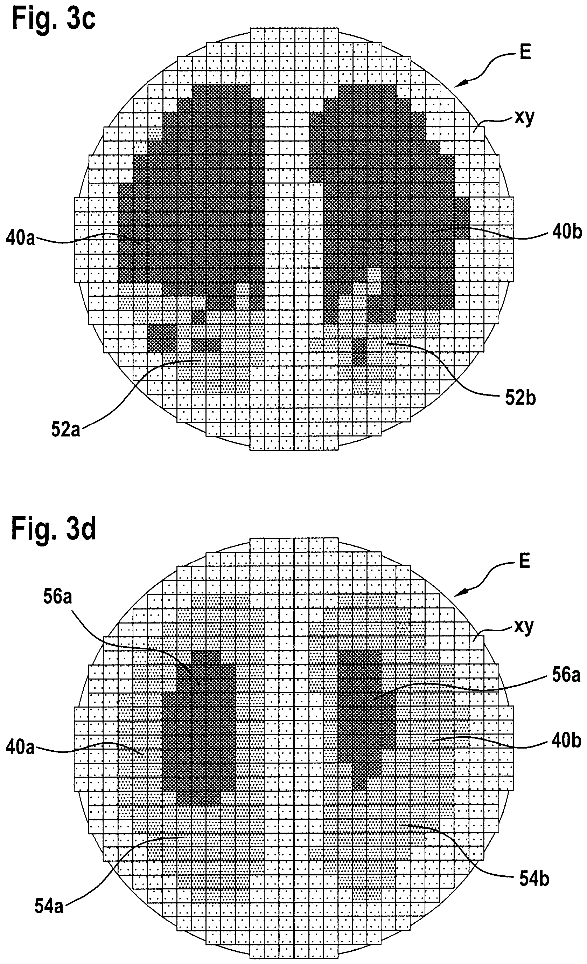

Allerdings deutet sich in Fig. 3a und 3b bereits an, dass im unteren zentralen Bereich 52a, 52b beider Lungenflügel 40a, 40b die Impedanzwerte sowohl am Ende Expiration als auch am Ende der Inspiration etwas geringer sind als bei der in Fig. 2a und 2b dargestellten gesunden Lunge. Betrachtet man das in Fig. 3c dargestellte Bild der Impedanzdifferenzen EIT_ei_xy - EIT_ee_xy, so fällt sofort auf, dass in den unteren zentralen Bereichen 52a, 52b beider Lungenflügel die Impedanzdifferenz unterhalb des gewählten Schwellenwerts (dieser beträgt in dem gewählten Beispiel das 0,5 fache der maximalen Impedanzdifferenz) liegt. Das deutet darauf hin, dass die Alveolen in diesem Bereich am Ende der Inspirationsphase nur wenig stärker mit Atemgas gefüllt sind als am Ende der Expirationsphase.

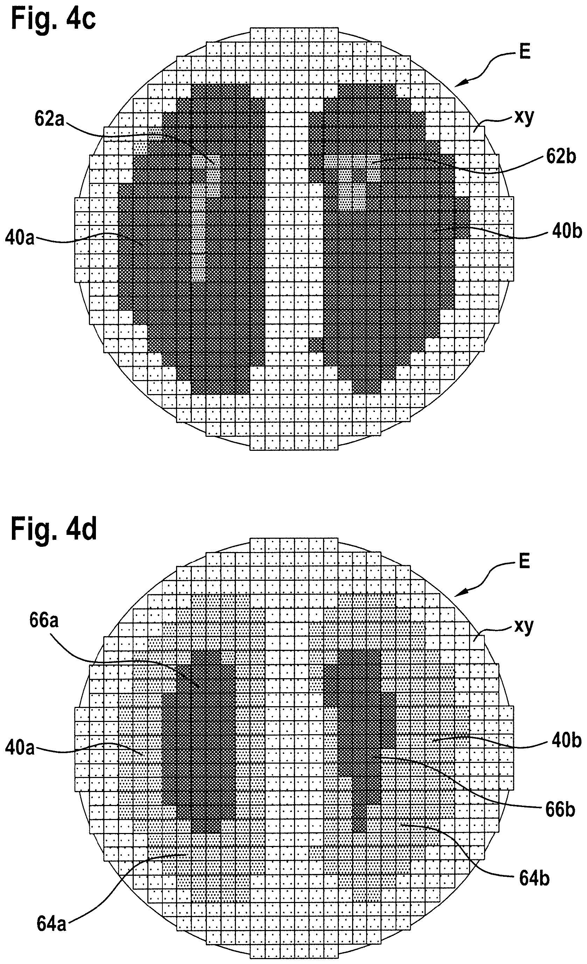

In Fig. 4a und 4b ist zu erahnen, dass im zentralen Bereich 62a, 62b beider Lungenflügel 40a, 40b die Impedanzwerte sowohl am Ende der Expiration als auch am Ende der Inspiration etwas höher sind als bei der in Fig. 2a und 2b dargestellten gesunden Lunge. Betrachtet man das in Fig. 4c dargestellte Bild der Impedanzdifferenzen EIT_ei_xy - EIT_ee_xy, so fällt sofort auf, dass auch hier in den zentralen Bereichen 62a, 62b beider Lungenflügel 40a, 40b die Impedanzdifferenz unterhalb des gewählten Schwellenwerts (dieser beträgt in dem gewählten Beispiel das 0,5 fache der maximalen Impedanzdifferenz) liegt. Das deutet darauf hin, dass die Alveolen in diesem Bereich sowohl am Ende der Inspirationsphase als auch am Ende der Expirationsphase in ähnlicher Weise mit Atemgas gefüllt sind.

Man kann also anhand einer Auswertung der Differenz zwischen der Impedanz am Ende der Inspirationsphase EIT_ei_xy und der Impedanz am Ende der Expirationsphase EIT_ee_xy für jedes EIT-Pixel xy unterscheiden, ob die diesem EIT-Pixel xy zugeordneten Alveolen im Wesentlichen gesund sind oder einer krankhaften Veränderung unterliegen. Diese Unterscheidung lässt sich selbst dann treffen, wenn die Lunge für ein und demselben Atmungszyklus sowohl Bereiche mit überdehnten Alveolen als auch Bereiche mit kollabierten Alveolen aufweist, da man in beiden Fällen (siehe Fig. 3c und 4c) denselben Schwellenwert zur Unterscheidung heranziehen kann. Man kann allerdings nicht unterscheiden, ob die Alveolen in den krankhaften Bereichen 52a, 52b, 62a, 62c während der Expirationsphase kollabiert sind oder während der Inspirationsphase überdehnt worden sind.

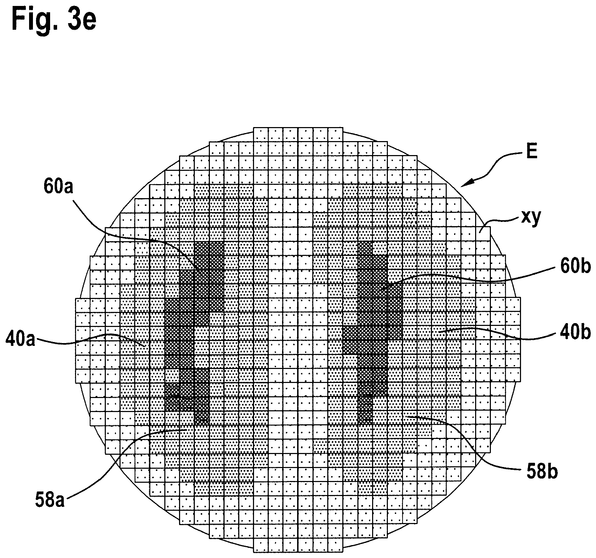

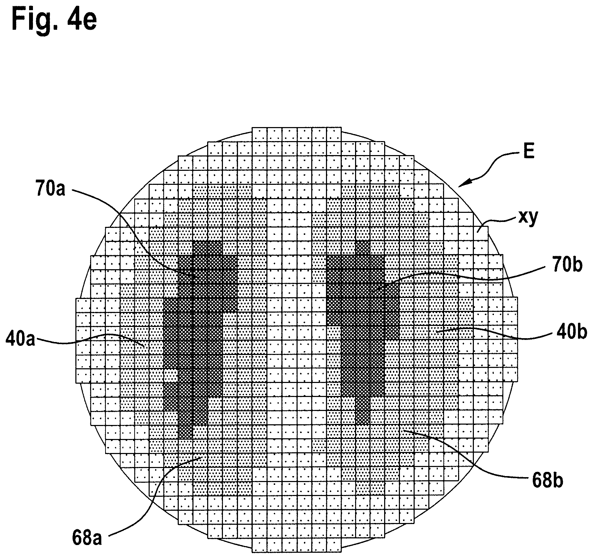

Betrachtet man allerdings die in den Fig. 3d, 3e bzw. Fig. 4d, 4e dargestellten binärisierten Werte der Impedanzen in der thorakalen Schnittebene E am Ende der Inspirationsphase bzw. am Ende der Expirationsphase, so erkennt man Folgendes:

Für EIT-Pixel xy in Bereichen 52a, 52b der Lungenflügel mit Alveolen, die während der Expirationsphase kollabiert sind, sind auch die Impedanzwerte am Ende der Expiration EIT_ee_xy niedrig. In Fig. 3e erkennt man das daran, dass sämtliche dieser EIT-Pixel zu den hell schraffierten Bereichen 58a, 58b gehören, also Impedanzwerte aufweisen, die innerhalb eines recht eng begrenzten Bereichs um den mininalen Impedanzwert am Ende der Expiration herum liegen. Daraus folgt: Alle EIT-Pixel xy die sowohl in der binärisierten Darstellung der Impedanzdifferenzen EIT_ei_xy - EIT_ee_xy gemäß Fig. 3c hell schattiert sind als auch in der binärisierten Darstellung der Impedanzen am Ende der Expiration EIT_ee_xy gemäß Fig. 3e hell schattiert sind, weisen in erheblichem Maße kollabierte Alveoloen auf. Beide Kriterien lassen sich recht einfach in einem automatisierten Beatmungssystem prüfen, so dass man ohne menschliche Eingriffe erkennen kann, ob die gewählte Einstellung der Beatmungsparameter korrigiert werden muss, weil in Teilen der Lunge Alveolen während der Expiration kollabieren. In solchen Fällen kann man beispielsweise vorsehen, dass das Beatmungssystem automatisch einen etwas höheren PEEP einstellt.

Für EIT-Pixel xy in Bereichen 62a, 62b der Lungenflügel mit Alveolen, die während der Inspirationsphase überdehnt worden sind, sind auch die Impedanzwerte am Ende der Inspiration EIT_ei_xy hoch. In Fig. 4d erkennt man das daran, dass sämtliche dieser EIT-Pixel zu den dunkel schraffierten Bereichen 66a, 66b gehören, also Impedanzwerte aufweisen, die in einem recht eng begrenzten Bereich um den maximalen Impedanzwert am Ende der Inspiration herum liegen. Daraus folgt: Alle EIT-Pixel xy die sowohl in der binärisierten Darstellung der Impedanzdifferenzen EIT_ei_xy - EIT_ee_xy gemäß Fig. 3c hell schattiert sind als auch in der binärisierten Darstellung der Impedanzwerte am Ende der Inspiration EIT_ei_xy gemäß Fig. 4d dunkel schraffiert sind, weisen in erheblichem Maße überdehnte Alveolen auf. Auch diese beiden Kriterien lassen sich recht einfach in einem automatisierten Beatmungssystem prüfen, so dass man ohne menschliche Eingriffe erkennen kann, ob die gewählte Einstellung der Beatmungsparameter korrigiert werden muss, weil in Teilen der Lunge Alveolen während der Inspiration überdehnt worden sind. In solchen Fällen kann man beispielsweise vorsehen, dass das Beatmungssystem automatisch einen etwas geringeren maximalen Beatmungsdruck Paw_max einstellt.

In Fällen, in denen während ein und desselben Atmungszyklus sowohl Bereiche in der Lunge auftreten, in denen Alveolen während der Inspirationsphase überdehnt werden, als auch Bereiche der Lunge auftreten, in denen Alveolen während der Expirationsphase kollabieren, lässt anhand der voneinander getrennten Apprüfung der beiden oben genannten Kumulativbedingungen auch ohne Weiteres eine Unterscheidung zwischen solchen Bereichen treffen. Als Folge des Auftretens von Lungenbereichen mit überdehnten Alveolen und von Lungenbereichen mit kollabierten Alveolen wird man einerseits den PEEP erhöhen, um dem Kollabieren von Alveolen entgegenzuwirken, und andererseits auch den maximalen Atemwegsdruck verringern, um dem Überdehnen von Alveolen entgegenzuwirken. Damit einher geht eine entsprechenden Verringerung des Tidaldrucks in den folgenden Atmungszyklen. Auch das lässt sich in eine automatisch arbeitenden Prozedur implementieren.

Damit schafft die vorliegende Erfindung die Möglichkeit einer weitgehend automatisch arbeitenden Beatmungseinrichtung, die in der Lage ist, wichtige Beatmungsdrücke wie den PEEP und/oder den maximalen Beatmungsdruck ohne Eingriffe von Ärzten oder Pflegepersonal anzupassen, wenn sich physiologische Parameter ändern.

Zur Einstellung bzw. Nachführung des PEEP kann man beispielsweise so vorgehen, dass man ausgehend von einem relativ geringen Startwert für den PEEP nach jedem Atmungszyklus, bzw. nach einer bestimmten Anzahl von Atmungszyklen die jeweils aufgenommenen EIT-Daten in der oben beschriebenen Weise auswertet und den Anteil von EIT-Pixeln xy bestimmt, für die beide der oben genannten Kriterien vorliegen, d.h. für die gilt:

$$\text{EIT\_ei\_xy} - \text{EIT\_ee\_xy} < k1 * \max(\text{EIT\_ei\_xy} - \text{EIT\_ee\_xy};$$

$$\text{für alle möglichen xy})$$

UND

$$\text{EIT\_ee\_xy} < k2 * \min(\text{EIT\_ee\_xy}; \text{für alle möglichen xy})$$

mit:

EIT_ei_xy: einem jeweiligen EIT Pixel zugeordneter elektrischer Impedanzwert am Ende der Inspirationsphase
EIT_ee_xy: einem jeweiligen EIT Pixel zugeordneter elektrischer Impedanzwert am Ende der Expirationsphase,
$0 \le k1 \le 1$, insbesondere $0{,}3 \le k1 \le 0{,}7$, insbesondere $0{,}4 \le k1 \le 0{,}6$, insbesondere $k1 = 0.5$;
$k2 \ge 1$, insbesondere $1{,}0 \le k2 \le 1{,}6$, insbesondere $1{,}2 \le k2 \le 1{,}4$, insbesondere $k2 = 1{,}3$.

Wenn der Anteil von EIT-Pixeln xy, für die der Vergleich positiv ist, an der Gesamtzahl der EIT-Pixel xy einen vorbestimmten ersten Schwellenwert k5 übersteigt, erhöht die Beatmungseinrichtung den Wert des PEEP für die nachfolgenden Atmungszyklen um einen vorbestimmten Betrag. Der erste Schwellenwert k5 kann beispielsweise in einem Bereich von 5 bis 25 % liegen, insbesondere 10 % betragen. Der PEEP kann beispielsweise um jeweils 1 cm $H_2O$ erhöht werden, wenn der erste Schwellenwert k5 überschritten wird. Als Ausgangswert für den PEEP empfiehlt sich ein moderater Wert, der noch Spielraum zur Einstellung nach oben lässt, beispielsweise 10 cm $H_2O$.-

Dieser Vergleich wird dann in vorbestimmten zeitlichen Abständen wiederholt. Der PEEP für die jeweils nachfolgenden Atmungszyklen wird solange erhöht, bis der Anteil von EIT-Pixeln xy, für die der Vergleich positiv ist, an der Gesamtzahl der EIT-Pixel yx unterhalb des ersten Schwellenwerts k5 liegt. Auch danach wird der Vergleich in vorbestimmten zeitlichen Abständen wiederholt. Solange der Anteil an EIT-Pixeln, für die der Vergleich positiv ist, unterhalb des ersten Schwellenwerts k5 liegt, bleibt der PEEP unverändert. Ergibt einer der Vergleiche erneut einen Anteil von EIT-Pixeln xy, für die der Vergleich positiv ist, von k5 oder größer, wird der PEEP erneut erhöht.

Der Wert des ersten Schwellenwerts k5 kann mit zunehmendem Wert des eingestellten PEEP größer gewählt werden. Damit wird verhindert, dass das Beatmungssystem den PEEP immer weiter erhöht, wenn sich der Anteil an EIT-Pixeln xy mit kollabierten Alveolen in Reaktion auf eine Erhöhung des PEEP nicht verändert. In dem oben genannten Beispiel einer Erhöhung des PEEP um jeweils 1 cm $H_2O$ pro Schritt, ausgehend von einem Anfangswert von 10 cm $H_2O$ für den PEEP, kann man beispielsweise bei jeder Erhöhung des PEEP den Wert des ersten Schwellenwerts k5 um 0,3 bis 3 %, insbesondere um 1 %, erhöhen.

Zur automatisierten Einstellung bzw. Nachführung eines maximalen Atemwegsdrucks Paw_max kann beispielsweise so vorgehen, dass man ausgehend von einem relativ hohen Startwert für den maximalen Atemwegsdruck Paw_max nach jedem Atmungszyklus, bzw. nach einer bestimmten Anzahl von Atmungszyklen, die jeweils aufgenommenen EIT-Daten in der oben beschriebenen Weise auswertet und den Anteil von EIT-Pixeln xy bestimmt, für den die folgenden beiden Bedingungen erfüllt sind:

$$\text{EIT\_ei\_xy} - \text{EIT\_ee\_xy} < k3 * \max(\text{EIT\_ei\_xy} - \text{EIT\_ee\_xy};$$

$$\text{für alle möglichen xy})$$

UND

$$\text{EIT\_ei\_xy} > k4 * \max(\text{EIT\_ei\_xy}; \text{für alle möglichen xy})$$

mit:

EIT_ei_xy: einem jeweiligen EIT Pixel zugeordneter elektrischer Impedanzwert am Ende der Inspirationsphase
EIT_ee_xy: einem jeweiligen EIT Pixel zugeordneter elektrischer Impedanzwert am Ende der Expirationsphase,
$0 \le k3 \le 1$, insbesondere $0{,}3 \le k3 \le 0{,}7$, insbesondere $0{,}4 \le k3 \le 0{,}6$, insbesondere $k3 = 0{,}5$;
$0 \le k4 \le 1$, insbesondere $0{,}5 \le k4 \le 0{,}9$, insbesondere $0{,}6 \le k4 \le 0{,}8$, insbesondere $k4 = 0{,}7$.

Wenn der Anteil von EIT-Pixeln xy, für die der Vergleich positiv ist, an der Gesamtzahl der EIT-Pixel xy einen vorbestimmten zweiten Schwellenwert k6 übersteigt, verringert die Beatmungseinrichtung den Wert des maximalen Atemwegsdrucks Paw_max für die nachfolgenden Atmungszyklen um einen vorbestimmten Betrag. Der erste Schwellenwert k6 kann beispielsweise in einem Bereich von 5 bis 25 % liegen, insbesondere 10 % betragen. Der maximale Atemwegsdruck Paw_max kann beispielsweise um jeweils 1 cm $H_2O$ verringert werden, wenn der zweite Schwellenwert k6 überschritten wird. Als Ausgangswert für den maximalen Atemwegsdruck Paw_max empfiehlt sich ein eher hoher Wert, beispielsweise 40 cm $H_2O$.

Dieser Vergleich wird dann in vorbestimmten zeitlichen Abständen wiederholt. Der maximale Atemwegsdruck Paw_max für die jeweils nachfolgenden Atmungszyklen wird solange verringert, bis der Anteil von EIT-Pixeln xy, für die der Vergleich positiv ist, an der Gesamtzahl der EIT-Pixel xy unterhalb des zweiten Schwellenwerts k6 liegt. Auch danach wird der Vergleich in vorbestimmten zeitlichen Abständen wiederholt. Solange der Anteil an EIT-Pixeln, für die der Vergleich positiv ist, unterhalb des zweiten Schwellenwerts k6 liegt, bleibt der Atemwegsdruck Paw_max unverändert. Ergibt einer der Vergleiche erneut einen Anteil von EIT-Pixeln xy, für die der Vergleich positiv ist, von k6 oder größer, wird der Atemwegsdruck Paw_max erneut verringert.

Der Wert des zweiten Schwellenwerts k6 kann mit abnehmendem Wert des eingestellten maximalen Atemwegsdrucks Paw_max größer gewählt werden. Damit wird verhindert, dass das Beatmungssystem den maximalen Atemwegsdrucks Paw_max immer weiter verringert, wenn sich der Anteil an EIT-Pixeln xy mit überdehnten Alveolen in Reaktion auf eine Verringerung des maximalen Atemwegsdrucks Paw_max nicht verändert. In dem oben genannten Beispiel einer Verringerung des maximalen Atemwegsdrucks Paw_max um jeweils 1 cm $H_2O$ pro Schritt, ausgehend von einem Anfangswert von 40 cm $H_2O$ für den maximalen Atemwegsdrucks Paw_max, kann man beispielsweise bei jeder Verringerung von Paw_max den Wert des zweiten Schwellenwerts k6 um 0,3 bis 3 %, insbesondere um 1 %, erhöhen.

## Patentansprüche

1. System zur automatisierten Einstellung eines durch eine Beatmungseinrichtung (100) vorgegebenen Drucks, umfassend

   eine Anordnung für elektrische Impedanztomographie (20) zur Erfassung einer elektrischen Impedanzverteilung (EIT_ei_xy, EIT_ee_xy) entlang wenigstens eines zweidimensionalen Schnitts (E) durch den menschlichen Thorax wenigstens am Ende einer Inspirationsphase und am Ende einer zugeordneten Expirationsphase;
   eine Einrichtung zur Unterteilung der erfassten elektrischen Impedanzverteilung am Ende der Inspirationsphase (EIT_ei_xy) und am Ende der Expirationsphase (EIT_ee_xy) in eine Mehrzahl von EIT-Pixeln (xy) und zur Bestimmung eines einem jeweiligen EIT-Pixel (xy) zugeordneten Werts der elektrischen Impedanz am Ende der Inspirationsphase (EIT_ei_xy) und am Ende der Expirationsphase (EIT_ee_xy); und
   eine Einrichtung zur automatisierten Einstellung des durch die Beatmungseinrichtung (100) vorgegebenen Drucks auf Grundlage eines Vergleichs

   (i) eines Unterschieds zwischen dem einem einzelnen EIT-Pixel (xy) zugeordneten Wert der elektrischen Impedanz am Ende der Inspirationsphase (EIT_ei_xy) und dem dem jeweiligen einzelnen EIT-Pixel (xy) zugeordneten Wert der elektrischen Impedanz am Ende der Expirationsphase (EIT_ee_xy)
   (ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz und am Ende der Expirationsphase;

   wobei der Vergleich als "ja/nein"-Test ausgestaltet ist, derart dass anhand des Vergleichs geprüft wird, ob eine vorbestimmte Bedingung erfüllt ist oder nicht.

2. System nach Anspruch 1, wobei der Unterschied zwischen dem einem einzelnen EIT-Pixel (xy) zugeordneten Wert der elektrischen Impedanz am Ende der Inspirationsphase (EIT_ei_xy) und dem dem jeweiligen einzelnen EIT-Pixel (xy) zugeordneten Wert der elektrischen Impedanz am Ende der Expirationsphase (EIT_ee_xy) und/oder der auf Grundlage der Gesamtheit der EIT-Pixel bestimmte Unterschied zwischen dem Wert der elektrischen Impedanz am Ende der Inspirationsphase und dem Wert der elektrischen Impedanz und am Ende der Expirationsphase als eine Differenz zwischen den jeweiligen Werten der elektrischen Impedanz am Ende der Inspirationsphase und der elektrischen Impedanz am Ende der Expirationsphase definiert ist.

3. System nach Anspruch 1, wobei der Unterschied zwischen dem einem einzelnen EIT-Pixel (xy) zugeordneten Wert der elektrischen Impedanz am Ende der Inspirationsphase (EIT_ei_xy) und dem dem jeweiligen einzelnen EIT-Pixel (xy) zugeordneten Wert der elektrischen Impedanz am Ende der Expirationsphase (EIT_ee_xy) und/oder der auf Grundlage der Gesamtheit der EIT-Pixel bestimmte Unterschied zwischen dem Wert der elektrischen Impedanz am Ende der Inspirationsphase und dem Wert der elektrischen Impedanz und am Ende der Expirationsphase als ein Verhältnis zwischen den jeweiligen Werten der elektrischen Impedanz am Ende der Inspirationsphase und der elektrischen Impedanz am Ende der Expirationsphase definiert ist.

4. System nach einem der Ansprüche 1 bis 3, wobei der auf Grundlage der Gesamtheit der EIT-Pixel bestimmte Unterschied zwischen dem Wert der elektrischen Impedanz am Ende der Inspirationsphase und dem Wert der elektrischen Impedanz am Ende der Expirationsphase ein Maximalwert aller Unterschiede zwischen dem Wert der elektrischen Impedanz am Ende der Inspirationsphase (EIT_ei_xy) für eines der EIT-Pixel und dem Wert der elektrischen Impedanz am Ende der Expirationsphase (EIT_ee_xy) für dieses EIT-Pixel ist.

5. System nach einem der Ansprüche 1 bis 4, wobei die Einrichtung zur automatisierten Einstellung des durch die Beatmungseinrichtung (100) vorgegebenen Drucks den einem jeweiligen EIT-Pixel (xy) zugeordneten Unterschied zwischen dem Wert der elektrischen Impedanz am Ende der Inspirationsphase (EIT_ei_xy) und dem Wert der elektrischen Impedanz am Ende der Expirationsphase (EIT_ee_xy) auch mit dem jeweils auf Grundlage der Gesamtheit der EIT-Pixel bestimmten Wert der elektrischen Impedanz am Ende der Inspirationsphase und/oder am Ende der Expirationsphase vergleicht.

6. System nach einem der Ansprüche 1 bis 5, wobei die Einrichtung zur automatisierten Einstellung des durch die Beatmungseinrichtung (100) vorgegebenen Drucks anhand des Vergleichs diejenigen EIT-Pixel (xy) bestimmt, die eine vorgegebene Bedingung erfüllen, und dann eine Korrektur des vorgegebenen Drucks vornimmt, wenn der Anteil der EIT-Pixel, die die Bedingung erfüllen, größer ist als ein vorgegebener Schwellenwert (k5, k6).

7. System nach einem der Ansprüche 1 bis 6, umfassend eine Einrichtung zur automatisierten Einstellung eines durch die Beatmungseinrichtung vorgegebenen positiven endexpiratorischen Drucks (PEEP) auf Grundlage eines Vergleichs

   (i) eines Unterschieds zwischen dem einem einzelnen EIT-Pixel (xy) zugeordneten Wert der elektrischen Impedanz am Ende der Inspirationsphase (EIT_ei_xy) und dem dem jeweiligen einzelnen EIT-Pixel (xy) zugeordneten Wert der elektrischen Impedanz am Ende der Expirationsphase (EIT_ee_xy) einerseits,
   (ii) mit einem auf Grundlage der Gesamtheit der EIT-Pixel bestimmten Unterschied zwischen dem Wert der elektrischen Impedanz am Ende der Inspirationsphase (EIT_ei_gesamt) und dem Wert der elektrischen Impedanz am Ende der Expirationsphase sowie mit dem auf Grundlage der Gesamtheit der EIT-Pixel bestimmten Wert der elektrischen Impedanz am Ende der Expirationsphase andererseits.

8. System nach Anspruch 7, wobei der auf Grundlage der Gesamtheit der EIT-Pixel bestimmte Wert der elektrischen Impedanz am Ende der Expirationsphase ein Minimalwert aller Werte der elektrischen Impedanz am Ende der Expirationsphase (EIT_ee_min) für ein jeweiliges der EIT-Pixel ist.

9. System nach Anspruch 7 oder 8, wobei die Einrichtung zur automatisierten Einstellung eines durch die Beatmungseinrichtung (100) vorgegebenen positiven endexpiratorischen Drucks (PEEP) diejenigen EIT-Pixel (xy), für die der Vergleich positiv ist, Lungenbereichen mit kollabierten Alveolen (52a/58a, 52b/58b) zuordnet.

10. System nach Anspruch 9, wobei die Zuordnung von EIT-Pixeln (xy) zu Lungenbereichen mit kollabierten Alveolen (52a/58a, 52b/58b) dann erfolgt, wenn die folgenden beiden Bedingungen erfüllt sind:

$$EIT\_ei\_xy - EIT\_ee\_xy < k1 * max(EIT\_ei\_xy - EIT\_ee\_xy;$$
$$für\ alle\ möglichen\ xy)$$

UND

$$EIT\_ee\_xy < k2 * min(EIT\_ee\_xy;\ für\ alle\ möglichen\ xy)$$

mit:

EIT_ei_xy:

einem jeweiligen EIT Pixel
zugeordneter elektrischer Impedanzwert
am Ende der Inspirationsphase

EIT_ee_xy:

einem jeweiligen EIT Pixel
zugeordneter elektrischer Impedanzwert
am Ende der Expirationsphase,

$0 \leq k1 \leq 1$,

insbesondere $0{,}3 \leq k1 \leq 0{,}7$,
insbesondere $0{,}4 \leq k1 \leq 0{,}6$,
insbesondere $k1 = 0.5$;

$k2 \geq 1$,

insbesondere $1{,}0 \leq k2 \leq 1{,}6$,
insbesondere $1{,}2 \leq k2 \leq 1{,}4$,
insbesondere $k2 = 1{,}3$.

11. System nach einem der Ansprüche 7 bis 10, wobei die Einrichtung zur automatisierten Einstellung eines durch die Beatmungseinrichtung (100) vorgegebenen positiven endexpiratorischen Drucks (PEEP) dann, wenn der Anteil von EIT-Pixeln (xy), für die der Vergleich positiv ist, an der Gesamtzahl der EIT-Pixel (xy) einen vorbestimmten ersten Schwellenwert (k5) übersteigt, den Wert des positiven endexpiratorischen Drucks (PEEP) für die nachfolgenden Atmungszyklen um einen vorbestimmten Betrag erhöht.

12. System nach einem der Ansprüche 7 bis 11, wobei der Vergleich in vorbestimmten zeitlichen Abständen wiederholt wird und der positive endexpiratorische Druck für die nachfolgenden Atmungszyklen (PEEP) solange erhöht wird, bis der Anteil von EIT-Pixeln (xy), für die der Vergleich positiv ist, an der Gesamtzahl der EIT-Pixel unterhalb des ersten Schwellenwerts (k5) liegt.

13. System nach Anspruch 11 oder 12, wobei der Wert des ersten Schwellenwerts (k5) mit zunehmendem Wert des positiven endexpiratorischen Drucks (PEEP) größer gewählt wird.

14. System nach einem der Ansprüche 1 bis 13, umfassend eine Einrichtung zur automatisierten Einstellung eines durch die Beatmungseinrichtung (100) vorgegebenen maximalen Atemwegsdrucks (Paw_max) auf Grundlage eines Vergleichs

(i) eines Unterschieds zwischen dem einem einzelnen EIT-Pixel (xy) zugeordneten Wert der elektrischen Impedanz am Ende der Inspirationsphase (EIT_ei_xy) und dem dem jeweiligen einzelnen EIT-Pixel (xy) zugeordneten Wert der elektrischen Impedanz am Ende der Expirationsphase (EIT_ee_xy) einerseits,
(ii) mit einem auf Grundlage der Gesamtheit der EIT-Pixel bestimmten Unterschied zwischen dem Wert der elektrischen Impedanz am Ende der Inspirationsphase und dem Wert der elektrischen Impedanz und am Ende der Expirationsphase sowie mit dem auf Grundlage der Gesamtheit der EIT-Pixel bestimmten Wert der elektrischen Impedanz am Ende der Inspirationsphase andererseits.

15. System nach Anspruch 14, wobei der auf Grundlage der Gesamtheit der EIT-Pixel bestimmte Wert der elektrischen Impedanz am Ende der Inspirationsphase ein Maximalwert aller Werte der elektrischen Impedanz am Ende der Inspirationsphase (EIT_ei_xy) für ein jeweiliges der EIT-Pixel (xy) ist.

16. System nach Anspruch 14 oder 15, wobei die Einrichtung zur automatisierten Einstellung eines durch die Beatmungseinrichtung (100) vorgegebenen maximalen Atemwegsdrucks (Paw_max) diejenigen EIT-Pixel (xy), für die der Vergleich positiv ist, Lungenbereichen mit überdehnten Alveolen (62a/66a; 62b/66b) zuordnet.

17. System nach Anspruch 16, wobei die Zuordnung von EIT-Pixeln (xy) zu Lungenbereichen mit überdehnten Alveolen (62a/66a; 62b/66b) dann erfolgt, wenn die folgenden beiden Bedingungen erfüllt sind:

$$\text{EIT\_ei\_xy} - \text{EIT\_ee\_xy} < \text{k3} * \max(\text{EIT\_ei\_xy} - \text{EIT\_ee\_xy};$$

$$\text{für alle möglichen xy})$$

UND

$$\text{EIT\_ei\_xy} > \text{k4} * \max(\text{EIT\_ei\_xy}; \text{für alle möglichen xy})$$

mit:

EIT_ei_xy:

einem jeweiligen EIT Pixel
zugeordneter elektrischer Impedanzwert
am Ende der Inspirationsphase

EIT_ee_xy:

einem jeweiligen EIT Pixel
zugeordneter elektrischer Impedanzwert
am Ende der Expirationsphase,

$0 \leq \text{k3} \leq 1$,

insbesondere $0{,}3 \leq \text{k3} \leq 0{,}7$,
insbesondere $0{,}4 \leq \text{k3} \leq 0{,}6$,
insbesondere k3 = 0,5;

$0 \leq \text{k4} \leq 1$,

insbesondere $0{,}5 \leq \text{k4} \leq 0{,}9$,
insbesondere $0{,}6 \leq \text{k4} \leq 0{,}8$,
insbesondere k4 = 0,7.

18. System nach einem der Ansprüche 14 bis 17, wobei die Einrichtung zur automatisierten Einstellung eines durch die Beatmungseinrichtung (100) vorgegebenen maximalen Atemwegsdrucks (Paw_max) dann, wenn der Anteil von EIT-Pixeln (xy), für die der Vergleich positiv ist, an der Gesamtzahl der EIT-Pixel (xy) einen vorbestimmten zweiten Schwellenwert (k6) übersteigt, den Wert des maximalen Atemwegsdrucks (Paw_max) für die nachfolgenden Atmungszyklen um einen vorbestimmten Betrag verringert.

19. System nach einem der Ansprüche 14 bis 18, wobei der Vergleich in vorbestimmten zeitlichen Abständen wiederholt wird und der maximale Atemwegsdruck (Paw_max) für die nachfolgenden Atmungszyklen solange verringert wird, bis der Anteil von EIT-Pixeln (xy), für die der Vergleich positiv ist, an der der Gesamtzahl der EIT-Pixel unterhalb des zweiten Schwellenwerts (k6) liegt.

20. System nach Anspruch 18 oder 19, wobei der Wert des zweiten Schwellenwerts (k6) mit abnehmendem Wert des maximalen Atemwegsdrucks (Paw_max) größer gewählt wird.

21. System nach einem der Ansprüche 1 bis 20, wobei die Anordnung für elektrische Impedanztomographie (20) eine elektrische Impedanzverteilung in Form von EIT-Pixeln (xy), die in einem vorgegebenen Raster angeordnet sind, zugeordneten elektrischen Impedanzwerten erfasst und wobei die Einrichtung zur Unterteilung der erfassten elektrischen Impedanzverteilung in eine Mehrzahl von EIT-Pixeln (xy) einem jeweiligen Rasterelement genau den jeweiligen Wert der elektrischen Impedanz zuordnet.

22. System nach einem der Ansprüche 1 bis 21, wobei die Anordnung für elektrische Impedanztomographie (20) eine elektrische Impedanzverteilung in Form von EIT-Elementarpixeln, die in einem vorgegebenen Raster angeordnet sind, zugeordneten elektrischen Impedanzwerten erfasst und wobei die Einrichtung zur Unterteilung der erfassten elektrischen Impedanzverteilung in eine Mehrzahl von EIT-Pixeln (xy) einen jeweiligen der EIT-Elementarpixel genau einem EIT-Pixel (xy) zuordnet und für jeden der EIT-Pixel (xy) einen zugeordneten Wert der elektrischen Impedanz bestimmt.

23. System nach einem der Ansprüche 1 bis 22, ferner umfassend eine Filtereinrichtung zum Ausfiltern solcher zeitlich schnell veränderlicher Anteile aus den erfassten elektrischen Impedanzwerten, die auf kardiale Einflüsse zurückzuführen sind.

24. Beatmungseinrichtung (100), umfassend ein System zur automatischen Einstellung eines von der Beatmungseinrichtung vorgegebenen Drucks nach einem der vorangehenden Ansprüche.

**Claims**

1. A system for automated adjustment of a pressure set by a respiration device (100), comprising

    an arrangement for electrical impedance tomography (20) for detecting an electrical impedance distribution (EIT_ei_xy, EIT_ee_xy) along at least a two-dimensional cross-section (E) through the human thorax at least at the end of an inspiration phase and at the end of an associated expiration phase;
    a device for dividing the detected electrical impedance distribution at the end of the inspiration phase (EIT_ei_xy) and at the end of the expiration phase (EIT_ee_xy) into a plurality of EIT pixels (xy) and for determining a value of the electrical impedance at the end of the inspiration phase (EIT_ei_xy) and at the end of the expiration phase (EIT_ee_xy), as associated with a respective EIT pixel; and
    a device for automated adjustment of the pressure set by the respiration device (100) on the basis of a comparison

        (i) of a deviation between the value of the electrical impedance at the end of the inspiration phase (EIT_ei_xy) associated with an individual EIT pixel (xy) and the value of the electrical impedance at the end of the expiration phase (EIT_ee_xy) associated with the respective individual EIT pixel
        (ii) with a deviation between the value of the electrical impedance at the end of the inspiration phase and the value of the electrical impedance at the end of the expiration phase, as determined on the basis of the entirety of the EIT pixels;

    wherein the comparison is in the form of a "Yes/No" test such that it is checked by way of the comparison whether or not a predetermined condition is fulfilled.

2. The system of claim 1,
    wherein the deviation between the value of the electrical impedance at the end of the inspiration phase (EIT_ei_xy) associated with an individual EIT pixel (xy) and the value of the electrical impedance at the end of the expiration phase (EIT_ee_xy) associated with the respective individual EIT pixel (xy) and/or the deviation between the value of the electrical impedance at the end of the inspiration phase and the value of the electrical impedance at the end of the expiration phase, as determined on the basis of the entirety of the EIT pixels, is defined as a difference between the respective values of the electrical impedance at the end of the inspiration phase and the electrical impedance at the end of the expiration phase.

3. The system of claim 1,
    wherein the deviation between the value of the electrical impedance at the end of the inspiration phase (EIT_ei_xy) associated with an individual EIT pixel (xy) and the value of the electrical impedance at the end of the expiration phase (EIT_ee_xy) associated with the respective individual EIT pixel (xy) and/or the deviation between the value of the electrical impedance at the end of the inspiration phase and the value of the electrical impedance at the end of the expiration phase, as determined on the basis of the entirety of the EIT pixels, is defined as a ratio between the respective values of the electrical impedance at the end of the inspiration phase and the electrical impedance at the end of the expiration phase.

4. The system of any of claims 1 to 3,
    wherein the deviation between the value of the electrical impedance at the end of the inspiration phase and the value of the electrical impedance at the end of the expiration phase, as determined on the basis of the entirety of the EIT pixels, is a maximum value of all differences between the values of the electrical impedance at the end of the inspiration phase (EIT_ei_xy) for respective ones of the EIT pixels and the values of the electrical impedance at the end of the expiration phase (EIT_ee_xy) for said respective ones of the EIT pixel.

5. The system of any of claims 1 to 4,
wherein the device for automated adjustment of the pressure set by the respiration device (100) compares the deviation between the value of the electrical impedance at the end of the inspiration phase (EIT_ei_xy) and the value of the electrical impedance at the end of the expiration phase (EIT_ee_xy), as associated with a respective EIT pixel (xy), also with the value of the electrical impedance at the end of the inspiration phase and/or at the end of the expiration phase, as respectively determined on the basis of the entirety of the EIT pixels.

6. The system of any of claims 1 to 5,
wherein the device for automated adjustment of the pressure set by the respiration device (100), by way of the comparison, determines those EIT pixels (xy) that fulfill a predetermined condition, and effects a correction of the set pressure when the percentage of EIT pixels fulfilling said condition is greater than a predetermined threshold value (k5, k6).

7. The system of any of claims 1 to 6,
comprising a device for automated adjustment of a positive end-expiratory pressure (PEEP) set by the respiration device on the basis of a comparison

(i) of a deviation between the value of the electrical impedance at the end of the inspiration phase (EIT_ei_xy) associated with an individual EIT pixel (xy) and the value of the electrical impedance at the end of the expiration phase (EIT_ee_xy) associated with the respective individual EIT pixel (xy), on the one hand,
(ii) with a deviation between the value of the electrical impedance at the end of the inspiration phase (EIT_ei_gesamt) and the value of the electrical impedance at the end of the expiration phase, as determined on the basis of the entirety of the EIT pixels, and the value of the electrical impedance at the end of the expiration phase as determined on the basis of the entirety of the EIT pixels, on the other hand.

8. The system of claim 7,
wherein the value of the electrical impedance at the end of the expiration phase determined on the basis of the entirety of the EIT pixels is a minimum value of all values of the electrical impedance at the end of the expiration phase (EIT_ee_min) for respective ones of the EIT pixels.

9. The system of claim 7 or 8,
wherein the device for automated adjustment of a positive end-expiratory pressure (PEEP) set by the respiration device (100) associates those EIT pixels (xy) for which the comparison is positive with lung regions having collapsed alveoli (52a/58a, 52b/58b).

10. The system of claim 9,
wherein the association of EIT pixels (xy) with lung regions with collapsed alveoli (52a/58a, 52b/58b) is effected when the following two conditions are fulfilled:

$$EIT\_ei\_xy - EIT\_ee\_xy < k1 * max(EIT\_ei\_xy - EIT\_ee\_xy;$$
$$for\ all\ possible\ xy)$$

AND

$$EIT\_ee\_xy < k2 * min(EIT\_ee\_xy;\ for\ all\ possible\ xy)$$

wherein:

EIT_ei_xy: electrical impedance value at the end of the inspiration phase associated with a respective EIT pixel
EIT_ee_xy: electrical impedance value at the end of the

expiration phase associated with a respective EIT pixel,
$0 \leq k1 \leq 1$,

in particular $0.3 \leq k1 \leq 0.7$,
in particular $0.4 \leq k1 \leq 0.6$,
in particular k1 = 0.5;

$k2 \geq 1$,

in particular $1.0 \leq k2 \leq 1.6$,
in particular $1.2 \leq k2 \leq 1.4$,
in particular k2 = 1.3.

**11.** The system of any of claims 7 to 10,
wherein the device for automated adjustment of a positive end-expiratoy pressure (PEEP) set by the respiration device (100) increases the value of the positive end-expiratory pressure (PEEP) for the subsequent breathing cycles by a predetermined amount, when the percentage of EIT pixels (xy), for which the comparison is positive, exceeds a predetermined first threshold value (k5) in the total number of EIT pixels (xy).

**12.** The system of any of claims 7 to 11,
wherein the comparison is repeated in predetermined time intervals and the positive end-expiratory pressure (PEEP) for the subsequent breathing cycles is increased until the percentage of EIT pixels (xy), for which the comparison is positive, in the total number of EIT pixels is below the first threshold value (k5).

**13.** The system of claim 11 or 12,
wherein the value of the first threshold value (k5) is selected greater with an increasing value of the positive end-expiratory pressure (PEEP).

**14.** The system of any of claims 1 to 13,
comprising a device for automated adjustment of a maximum airway pressure (Paw_max) set by the respiration device (100), on the basis of a comparison

(i) of a deviation between the value of the electrical impedance at the end of the inspiration phase (EIT_ei_xy) associated with an individual EIT pixel (xy) and the value of the electrical impedance at the end of the expiration phase (EIT_ee_xy) associated with the respective individual EIT pixel (xy) on the one hand,
(ii) with a deviation between the value of the electrical impedance at the end of the inspiration phase and the value of the electrical impedance at the end of the expiration phase, as determined on the basis of the entirety of the EIT pixels, and the value of the electrical impedance at the end of the inspiration phase, as determined on the basis of the entirety of the EIT pixels, on the other hand.

**15.** The system of claim 14,
wherein the value of the electrical impedance at the end of the inspiration phase, as determined on the basis of the entirety of the EIT pixels, is a maximum value of all values of the electrical impedance at the end of the inspiration phase (EIT_ei_xy) for respective ones of the EIT pixels (xy).

**16.** The system of claim 14 or 15,
wherein the device for automated adjustment of a maximum airway pressure (Paw_max) set by the respiration device (100) associates those EIT pixels (xy) for which the comparison is positive with lung regions having overextended alveoli (62a/66a; 62b/66b).

**17.** The system of claim 16,
wherein the association of EIT pixels (xy) with lung regions having overextended alveoli (62a/66a; 62b/66b) is effected when the following two conditions are fulfilled:

$$\text{EIT\_ei\_xy} - \text{EIT\_ee\_xy} < k3 * \max(\text{EIT\_ei\_xy} - \text{EIT\_ee\_xy};$$
$$\text{for all possible xy)}$$

AND

$$EIT\_ei\_xy > k4 * max(EIT\_ei\_xy; \text{ for all possible } xy)$$

wherein:

EIT_ei_xy: electrical impedance value at the end of the
inspiration phase associated with a respective EIT pixel
EIT_ee_xy: electrical impedance value at the end of the
expiration phase associated with a respective EIT pixel
$0 \leq k3 \leq 1$,

in particular $0.3 \leq k3 \leq 0.7$,
in particular $0.4 \leq k3 \leq 0.6$,
in particular $k3 = 0.5$;

$0 \leq k4 \leq 1$,

in particular $0.5 \leq k4 \leq 0.9$,
in particular $0.6 \leq k4 \leq 0.8$,
in particular $k4 = 0.7$.

18. The system of any of claims 14 to 17,
wherein the device for automated adjustment of a maximum airway pressure (Paw_max) set by the respiration device (100) decreases the value of the maximum airway pressure (Paw_max) for the subsequent breathing cycles by a predetermined amount, when the percentage of EIT pixels (xy), for which the comparison is positive, exceeds a predetermined second threshold value (k6) in the total number of EIT pixels (xy).

19. The system of any of claims 14 to 18,
wherein the comparison is repeated in predetermined time intervals and the maximum airway pressure (Paw_max) for the subsequent breathing cycles is decreased until the percentage of EIT pixels (xy), for which the comparison is positive, in the total number of EIT pixels is below the second threshold value (k6).

20. The system of claim 18 to 19,
wherein the value of the second threshold value (k6) is selected greater with a decreasing value of the maximum airway pressure (Paw_max).

21. The system of any of claims 1 to 20,
wherein the arrangement for electrical impedance tomography (20) detects an electrical impedance distribution in the form of electrical impedance values associated with EIT pixels (xy) arranged in a predetermined grid, and wherein the device for dividing the detected electrical impedance distribution into a plurality of EIT pixels (xy) associates exactly the respective value of the electrical impedance with a respective grid element.

22. The system of any of claims 1 to 21,
wherein the arrangement for electrical impedance tomography (20) detects an electrical impedance distribution in the form of electrical impedance values associated with elementary EIT pixels (xy) arranged in a predetermined grid, and wherein the device for dividing the detected electrical impedance distribution into a plurality of EIT pixels (xy) associates a respective one of the EIT elementary pixels with exactly one EIT pixel (xy) and determines for each one of the EIT pixels (xy) an associated value of the electrical impedance.

23. The system of any of claims 1 to 22,
further comprising a filter device for filtering out such rapidly time-variable components from the detected electrical impedance values that are due to cardiac effects.

24. A respiration device (100), comprising a system for automatic adjustment of a pressure set by the respiration device in accordance with any of the preceding claims.

**Revendications**

1. Système destiné au réglage automatisé d'une pression prédéfinie par l'intermédiaire d'un équipement respiratoire (100), comprenant:

   un agencement pour une tomographie à impédance électrique (20) destiné à l'enregistrement d'une distribution d'impédances électriques (EIT_ei_xy, EIT_ee_xy) le long d'au moins une coupe en deux dimensions (E) à travers le thorax humain au moins à la fin d'une phase d'inspiration et à la fin d'une phase d'expiration correspondante ;
   un équipement destiné à la répartition de la distribution d'impédances électriques enregistrée à la fin de la phase d'inspiration (EIT_ei_xy) et à la fin de la phase d'expiration (EIT_ee_xy) en une multitude de pixels EIT (xy) et destiné à la détermination d'une valeur de l'impédance électrique attribuée à un pixel EIT (xy) respectif à la fin de la phase d'inspiration (EIT_ei_xy) et à la fin de la phase d'expiration (EIT_ee_xy) ; et
   un équipement destiné au réglage automatisé de la pression prédéfinie par l'intermédiaire de l'équipement respiratoire (100) sur base d'une comparaison

   (i) d'une différence entre la valeur attribuée à un pixel EIT (xy) individuel, de l'impédance électrique à la fin de la phase d'inspiration (EIT_ei_xy) et la valeur attribuée au pixel EIT (xy) individuel respectif, de l'impédance électrique à la fin de la phase d'expiration (EIT_ee_xy) ;
   (ii) à une différence déterminée sur base de la totalité des pixels EIT entre la valeur de l'impédance électrique à la fin de la phase d'inspiration et la valeur de l'impédance électrique à la fin de la phase d'expiration ;
   dans lequel la comparaison est conçue sous la forme d'un test « oui/non », d'une manière telle que l'on procède à une vérification, en se basant sur la comparaison, du fait de savoir si une condition qui a été prédéfinie est remplie ou non.

2. Système selon la revendication 1, dans lequel la différence entre la valeur attribuée à un pixel EIT (xy) individuel, de l'impédance électrique à la fin de la phase d'inspiration (EIT_ei_xy) et la valeur attribuée au pixel EIT (xy) individuel respectif, de l'impédance électrique à la fin de la phase d'expiration (EIT_ee_xy) et/ou la différence déterminée sur base de la totalité des pixels EIT entre la valeur de l'impédance électrique à la fin de la phase d'inspiration et la valeur de l'impédance électrique à la fin de la phase d'expiration est définie comme étant une différence entre les valeurs respectives de l'impédance électrique à la fin de la phase d'inspiration et de l'impédance électrique à la fin de la phase d'expiration.

3. Système selon la revendication 1, dans lequel la différence entre la valeur attribuée à un pixel EIT (xy) individuel, de l'impédance électrique à la fin de la phase d'inspiration (EIT_ei_xy) et la valeur attribuée au pixel EIT (xy) individuel respectif, de l'impédance électrique à la fin de la phase d'expiration (EIT_ee_xy) et/ou la différence déterminée sur base de la totalité des pixels EIT entre la valeur de l'impédance électrique à la fin de la phase d'inspiration et la valeur de l'impédance électrique à la fin de la phase d'expiration est définie comme étant un rapport entre les valeurs respectives de l'impédance électrique à la fin de la phase d'inspiration et de l'impédance électrique à la fin de la phase d'expiration.

4. Système selon l'une quelconque des revendications 1 à 3, dans lequel la différence déterminée sur base de la totalité des pixels EIT entre la valeur de l'impédance électrique à la fin de la phase d'inspiration et la valeur de l'impédance électrique à la fin de la phase d'expiration représente une valeur maximale de toutes les différences entre la valeur de l'impédance électrique à la fin de la phase d'inspiration (EIT_ei_xy) pour un des pixels EIT et la valeur de l'impédance électrique à la fin de la phase d'expiration (EIT_ee_xy) pour ce pixel EIT.

5. Système selon l'une quelconque des revendications 1 à 4, dans lequel l'équipement destiné au réglage automatisé de la pression prédéfinie par l'intermédiaire de l'équipement respiratoire (100) compare la différence attribuée à un pixel EIT (xy) respectif entre la valeur de l'impédance électrique à la fin de la phase d'inspiration (EIT_ei_xy) et la valeur de l'impédance électrique à la fin de la phase d'expiration (EIT_ee_xy) également à la valeur déterminée de manière respective sur base de la totalité des pixels EIT de l'impédance électrique à la fin de la phase d'inspiration et/ou à la fin de la phase d'expiration.

6. Système selon l'une quelconque des revendications 1 à 5, dans lequel l'équipement destiné au réglage automatisé de la pression prédéfinie par l'intermédiaire de l'équipement respiratoire (100) détermine, sur base de la comparaison, les pixels EIT (xy) qui remplissent une condition prédéfinie et procède ensuite à une correction de la pression prédéfinie lorsque la fraction des pixels EIT qui remplissent la condition est supérieure à une valeur seuil prédéfinie (k5, k6).

7. Système selon l'une quelconque des revendications 1 à 6, comprenant un équipement pour le réglage automatisé d'une pression positive en fin d'expiration (PEEP) prédéfinie par l'intermédiaire de l'équipement respiratoire, sur base d'une comparaison

(i) d'une différence entre la valeur attribuée à un pixel EIT (xy) individuel, de l'impédance électrique à la fin de la phase d'inspiration (EIT_ei_xy) et la valeur attribuée au pixel EIT (xy) individuel respectif, de l'impédance électrique à la fin de la phase d'expiration (EIT_ee_xy), d'une part ;

(ii) à une différence déterminée sur base de la totalité des pixels EIT entre la valeur de l'impédance électrique à la fin de la phase d'inspiration (EIT_ei_total) et la valeur de l'impédance électrique à la fin de la phase d'expiration, et à la valeur déterminée sur base de la totalité des pixels EIT de l'impédance électrique à la fin de la phase d'expiration, d'autre part.

8. Système selon la revendication 7, dans lequel la valeur déterminée sur base de la totalité des pixels EIT, de l'impédance électrique à la fin de la phase d'expiration représente une valeur minimale de toutes les valeurs de l'impédance électrique à la fin de la phase d'expiration (EIT_ee_min) pour un pixel respectif parmi les pixels EIT.

9. Système selon la revendication 7 ou 8, dans lequel l'équipement destiné au réglage automatisé d'une pression positive en fin d'expiration (PEEP) prédéfinie par l'intermédiaire de l'équipement respiratoire (100) attribue aux pixels EIT (xy), pour lesquels la comparaison est positive, des zones pulmonaires comprenant des alvéoles affaissées (52a/58a, 52b/58b).

10. Système selon la revendication 9, dans lequel l'attribution de pixels EIT (xy) à des zones pulmonaires comprenant des alvéoles affaissées (52a/58a, 52b/58b) intervient lorsque les deux conditions suivantes sont remplies :

$$EIT\_ei\_xy - EIT\_ee\_xy < k1 * max(EIT\_ei\_xy - EIT\_ee\_xy \text{ ; pour tous les xy possibles})$$

ET

$$EIT\_ee\_xy < k2 * min(EIT\_ee\_xy \text{ ; pour tous les xy possibles})$$

où

EIT_ei_xy représente une valeur d'impédance électrique attribuée à un pixel EIT respectif à la fin de la phase d'inspiration ;

EIT_ee_xy représente une valeur d'impédance électrique attribuée à un pixel EIT respectif à la fin de la phase d'expiration ;

$0 \leq k1 \leq 1$, en particulier $0,3 \leq k1 \leq 0,7$, en particulier $0,4 \leq k1 \leq 0,6$, en particulier $k1 = 0,5$ ;

$K2 \geq 1$, en particulier $1,0 \leq k2 \leq 1,6$, en particulier $1,2 \leq k2 \leq 1,4$, en particulier $k2 = 1,3$.

11. Système selon l'une quelconque des revendications 7 à 10, dans lequel l'équipement destiné au réglage automatisé d'une pression positive en fin d'expiration (PEEP) prédéfinie par l'intermédiaire de l'équipement respiratoire (100), lorsque la fraction de pixels EIT (xy), pour lesquels la comparaison est positive, par rapport au nombre total des pixels EIT (xy), dépasse vers le haut une première valeur seuil prédéfinie (k5), augmente d'une quantité prédéfinie la valeur de la pression positive en fin d'expiration (PEEP) pour les cycles de respiration suivants.

12. Système selon l'une quelconque des revendications 7 à 11, dans lequel on répète la comparaison à des intervalles de temps prédéfinis et on augmente la pression positive en fin d'expiration pour les cycles de respiration suivants (PEEP) jusqu'à ce que la fraction de pixels EIT (xy), pour lesquels la comparaison est positive, par rapport au nombre total des pixels EIT (xy), se situe en dessous de la première valeur seuil (k5).

13. Système selon la revendication 11 ou 12, dans lequel on choisit une valeur plus grande en ce qui concerne la première valeur seuil (k5) de manière proportionnelle à la valeur croissante de la pression positive en fin d'expiration (PEEP).

**14.** Système selon l'une quelconque des revendications 1 à 13, comprenant un équipement pour le réglage automatisé d'une pression maximale des voies respiratoires (Paw_max) prédéfinie par l'intermédiaire de l'équipement respiratoire (100), sur base d'une comparaison

    (i) d'une différence entre la valeur attribuée à un pixel EIT (xy) individuel, de l'impédance électrique à la fin de la phase d'inspiration (EIT_ei_xy) et la valeur attribuée au pixel EIT (xy) individuel respectif, de l'impédance électrique à la fin de la phase d'expiration (EIT_ee_xy), d'une part ;
    (ii) à une différence déterminée sur base de la totalité des pixels EIT entre la valeur de l'impédance électrique à la fin de la phase d'inspiration et la valeur de l'impédance électrique à la fin de la phase d'expiration, et à la valeur déterminée sur base de la totalité des pixels EIT de l'impédance électrique à la fin de la phase d'expiration, d'autre part.

**15.** Système selon la revendication 14, dans lequel la valeur déterminée sur base de la totalité des pixels EIT de l'impédance électrique à la fin de la phase d'inspiration représente une valeur maximale de toutes les valeurs de l'impédance électrique à la fin de la phase d'inspiration (EIT_ei_xy) pour un pixel respectif parmi les pixels EIT (xy).

**16.** Système selon la revendication 14 ou 15, dans lequel l'équipement destiné au réglage automatisé d'une pression maximale des voies respiratoires (Paw_max) prédéfinie par l'intermédiaire de l'équipement respiratoire (100) attribue aux pixels EIT (xy), pour lesquels la comparaison est positive, des zones pulmonaires comprenant des alvéoles en hyperextension (62a/66a, 62b/66b).

**17.** Système selon la revendication 16, dans lequel l'attribution de pixels EIT (xy) à des zones pulmonaires comprenant des alvéoles en hyperextension (62a/66a, 62b/66b) intervient lorsque les deux conditions suivantes sont remplies :

$$EIT\_ei\_xy - EIT\_ee\_xy < k3 * max(EIT\_ei\_xy - EIT\_ee\_xy \ ; \ pour\ tous\ les\ xy\ possibles)$$

ET

$$EIT\_ei\_xy < k4 * max(EIT\_ei\_xy \ ; \ pour\ tous\ les\ xy\ possibles)$$

où

    EIT_ei_xy représente une valeur d'impédance électrique attribuée à un pixel EIT respectif à la fin de la phase d'inspiration ;
    EIT_ee_xy représente une valeur d'impédance électrique attribuée à un pixel EIT respectif à la fin de la phase d'expiration ;
    $0 \le k3 \le 1$, en particulier $0,3 \le k3 \le 0,7$, en particulier $0,4 \le k3 \le 0,6$, en particulier $k3 = 0,5$ ;
    $0 \le K4 \ge 1$, en particulier $0,5 \le k4 \le 0,9$, en particulier $0,6 \le k4 \le 0,8$, en particulier $k4 = 0,7$.

**18.** Système selon l'une quelconque des revendications 14 à 17, dans lequel l'équipement destiné au réglage automatisé d'une pression maximale des voies respiratoires (Paw_max) prédéfinie par l'intermédiaire de l'équipement respiratoire (100), lorsque la fraction de pixels EIT (xy), pour lesquels la comparaison est positive, par rapport au nombre total des pixels EIT (xy), dépasse vers le haut une deuxième valeur seuil prédéfinie (k6), diminue d'une quantité prédéfinie la valeur de la pression maximale des voies respiratoires (Paw_max) pour les cycles de respiration suivants.

**19.** Système selon l'une quelconque des revendications 14 à 18, dans lequel on répète la comparaison à des intervalles de temps prédéfinis et on diminue la pression maximale des voies respiratoires (Paw_max) pour les cycles de respiration suivants jusqu'à ce que la fraction de pixels EIT (xy), pour lesquels la comparaison est positive, par rapport au nombre total des pixels EIT (xy), se situe en dessous de la deuxième valeur seuil (k6).

**20.** Système selon la revendication 18 ou 19, dans lequel on choisit une valeur plus grande en ce qui concerne la deuxième valeur seuil (k6) de manière proportionnelle à la valeur décroissante de la pression maximale des voies respiratoires (Paw_max).

21. Système selon l'une quelconque des revendications 1 à 20, dans lequel l'agencement destiné à une tomographie à impédance électrique (20) enregistre des valeurs d'impédances électriques attribuées à une distribution d'impédances électriques sous la forme de pixels EIT (xy) qui sont disposés en formant un réseau prédéfini, et dans lequel l'équipement destiné à la répartition de la distribution d'impédances électriques enregistrée en une multitude de pixels EIT (xy) attribue à un élément de réseau respectif précisément la valeur respective de l'impédance électrique.

22. Système selon l'une quelconque des revendications 1 à 21, dans lequel l'agencement destiné à une tomographie à impédance électrique (20) enregistre des valeurs d'impédances électriques attribuées à une distribution d'impédances électriques sous la forme de pixels élémentaires EIT (xy) qui sont disposés en formant un réseau prédéfini, et dans lequel l'équipement destiné à la répartition de la distribution d'impédances électriques enregistrée en une multitude de pixels EIT (xy) attribue un des pixels élémentaires EIT respectif précisément à un pixel EIT et détermine, pour chacun des pixels EIT (xy), une valeur correspondante de l'impédance électrique.

23. Système selon l'une quelconque des revendications 1 à 22, comprenant en outre un équipement de filtration pour éliminer par filtration des fractions qui se modifient rapidement dans le temps, à partir des valeurs d'impédances électriques enregistrées, qui sont dues à des influences cardiales.

24. Equipement respiratoire (100) comprenant un système destiné au réglage automatisé d'une pression prédéfinie par l'intermédiaire de l'équipement respiratoire selon l'une quelconque des revendications précédentes.

## Fig. 1

## Fig. 2a

## Fig. 2b

## Fig. 2c

## Fig. 2d

# Fig. 2e

# Fig. 3a

# Fig. 3b

## Fig. 3c

E

xy

40a

40b

52a

52b

## Fig. 3d

56a

E

xy

56a

40a

40b

54a

54b

## Fig. 3e

# Fig. 4a

# Fig. 4b

## Fig. 4c

## Fig. 4d

Fig. 4e

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6502984 B1
- EP 1593341 B1
- US 20100228143 A1
- US 2006260611 A1
- US 6501198 B1
- DE 10301202 B3
- DE 10301202 B1

**In der Beschreibung aufgeführte Nicht-Patentliteratur**

- The Acute Respiratory Distress Syndrome Network. *The New England Journal of Medicine,* 2000, vol. 342, 1301-1308
- **GRASSO et al.** *American Journal of Respiratory Critical Care,* 2007, vol. 176, 761-767
- **BROCHARD L.** *Critical Care,* 2006, vol. 10, 156-158
- **BIKKER, I. G. et al.** *Critical Care,* 2010, vol. 14 (3), R100
- **TANAKA, H. et al.** *American Journal of Respiratory and Critical Care,* 2004, vol. 169 (7), 791-800
- Absolute Electrical Impedance Tomography (aEIT) Guided Ventilation Therapy in Critical Care Patients: Simulations and Future Trends. **MOULOUD A DE-NAI et al.** IEEE TRANSACTIONS ON INFORMA-TION TECHNOLOGY IN BIOMEDI-CINE. IEEE SERVICE CENTER, 01. Mai 2010, vol. 14, 641-649